# EUROPÄISCHE PATENTANMELDUNG

(11) **EP 2 786 742 A1**
(43) Veröffentlichungstag der Anmeldung: **08.10.2014**
(21) Anmeldenummer: 13161919.9
(22) Anmeldetag: 02.04.2013
(51) Int. Cl.: A61K 8/60

(54) **Kosmetika enthaltend Rhamnolipide**

(71) Anmelder: Evonik Industries AG, 45128 Essen (DE)
(72) Erfinder: Brandt, Kathrin Daniela, 45326 Essen (DE); Hartung, Christian, 45133 Essen (DE)

(57) **Zusammenfassung**

Gegenstand der Erfindung sind Kosmetika enthaltend Rhamnolipide.

## Beschreibung

### Gebiet der Erfindung

Gegenstand der Erfindung sind Kosmetika enthaltend Rhamnolipide.

### Stand der Technik

Surfactants werden heutzutage im Wesentlichen basierend auf Basis petrochemischer Rohstoffe hergestellt. Die Nutzung von Surfactants auf Basis nachwachsender Rohstoffe ist aufgrund der absehbaren Verknappung petrochemischer Rohstoffe und zunehmender Nachfrage nach Produkten, die auf nachwachsenden Rohstoffen basieren bzw. biologisch abbaubar sind, eine entsprechende Alternative. Rhamnolipide bestehen aus ein (Monorhamnosyllipide) oder zwei Rhamnoseresten (Dirhamnosyllipide) und ein oder zwei 3-Hydroxyfettsäureresten (siehe Handbook of Hydrocarbon and Lipid Microbiology, 2010, Seiten 3037-51).
Aufgabe der Erfindung war es, ein Surfactant für Kosmetika bereitzustellen.

### Beschreibung der Erfindung

Überraschenderweise wurde gefunden, dass Rhamnolipide hervorragende Surfactants für kosmetische Anwendungen darstellen.

Gegenstand der vorliegenden Erfindung sind daher kosmetische Formulierungen enthaltend mindestens ein Rhamnolipid.

### Beispiele:

Die im Folgende gelisteten Formulierungen werden jeweils zweimal hergestellt: Einmal wird als Rhamnolipid eine Rhamnolipidmischung eingesetzt, die Mono-Rhamnolipide und Di-Rhmanolipide mit einer Fettsäureverteilung von Rha-Rha-C8-C10, Rha-Rha-C10-C8, Rha-C8-C10, Rha-C10-C8, , Rha-Rha-C10-C10, Rha-Rha-C10-C12:1, Rha-Rha-C12:1-C10, Rha-C10-C10, Rha-Rha-C10-C12, Rha-Rha-C12-C10, Rha-C10-C12:1, Rha-C12:1-C10, Rha-Rha-C12-C12:1, Rha-Rha-C12:1-C12, Rha-Rha-C10-C14:1, Rha-Rha-C14:1-C10, Rha-C10-C12, Rha-C12-C10 und Rha-Rha-C12-C12
enthält,
und einmal wird als Rhamnolipid eine Rhamnolipidmischung eingesetzt, die 81 Gew.-%diRL-C10C10,
10 Gew.-% diRL-C10C12,
8 Gew.-% diRL-C10C12:1 und
1 Gew.-% monoRL-C10C10
enthält,
so dass ingesamt 210 Formulierungen hergestellt werden.

### Formulierung 1: Volume and Body Shampoo

| | |
|---|---|
| Aqua | ad 100% |
| Sodium Lauryl Sulfate | 7,0% |
| Sodium Laureth Sulfate | 5,0% |
| Cocamidopropyl Betaine | 2,0% |
| Rhamnolipid | 2,0% |
| Sodium Citrate | 1,5% |
| Sodium Xylenesulfonate | 1,5% |
| Sodium Chloride | 1,4% |
| Citric Acid | ad pH 5,5 |
| Sodium Benzoate | 1,0% |
| Hydroxypropyl Methylcellulose | 1,0% |
| Tetrasodium EDTA | 0,8% |
| Butylphenyl Methylpropional | 0,5% |
| Panthenol | 0,5% |
| Panthenyl Ethyl Ether | 0,3% |
| Linalool | 0,1% |
| Hexyl Cinnamal | 0,1% |
| Limonene | 0,1% |
| Benzyl Salicylate | 0,2% |
| Magnesium Nitrate | 0,1% |
| Magnesium Chloride | 0,1% |
| Methylchloroisothiazolinone | 0,1% |
| Dyes | q.s. |

### Formulierung 2: Repair Shampoo

| | |
|---|---|
| Aqua | ad 100% |
| Sodium Laureth Sulfate | 7,0% |
| Sodium Lauryl Sulfate | 3,0% |
| Rhamnolipid | 3,0% |
| Glycol Distearate | 2,5% |
| Cocamidopropyl Betaine | 2,0% |
| Sodium Citrate | 1,8% |
| Cocamide MEA | 1,8% |
| Sodium Xylenesulfonate | 1,5% |
| Dimethicone | 1,5% |
| Citric Acid | ad pH 5,5 |
| Sodium Benzoate | 0,7% |
| Polyquaternium-76 | 0,5% |
| Sodium Chloride | 0,9% |
| Glycerin | 0,5% |
| Tetrasodium EDTA | 0,3% |
| Butylphenyl Methylpropional | 0,2% |
| Hexyl Cinnamal | 0,1% |
| Linalool | 0,1% |
| alpha-Isomethyl lonone | 0,1% |
| Geraniol | 0,2% |
| Hydrochloric Acid | 0,1% |
| Magnesium Nitrate | 0,1% |
| Methylchloroisothiazolinone | 0,1% |
| Magnesium Chloride | 0,1% |
| Methylisothiazolinone | 0,1% |
| Dyes | q.s. |

### Formulierung 3: Anti-Dandruff Shampoo

| | |
|---|---|
| Aqua | ad 100% |
| Sodium Laureth Sulfate | 6,0% |
| Sodium Lauryl Sulfate | 5,0% |
| Rhamnolipid | 4,0% |
| Cocamide MEA | 4,0% |
| Zinc Carbonate | 3,0% |
| Glycol Distearate | 2,5% |
| Dimethicone | 1,0% |
| Zinc Pyrithione | 1,0% |
| Sodium Chloride | 0,6% |
| Cetyl Alcohol | 0,8% |
| Guar Hydroxypropyltrimonium Chloride | 0,3% |
| Sodium Xylenesulfonate | 0,5% |
| Magnesium Sulfate | 0,7% |
| Sodium Benzoate | 0,7% |
| Ammonium Laureth Sulfate | 0,3% |
| Benzyl Alcohol | 0,2% |
| Methylchloroisothiazolinone | 0,1% |
| Methylisothiazolinone | 0,1% |
| Parfum, Dyes | q.s. |

### Formulierung 4: Strengthening Shampoo

| | |
|---|---|
| Water | ad 100% |
| Sodium Lauryl Sulfate | 6,0% |
| Sodium Laureth Sulfate | 4,0% |
| Cocamidopropyl Betaine | 3,0% |
| Rhamnolipid | 3,0% |
| Glycol Distearate | 2,5% |
| Dimethicone | 2,0% |
| Sodium Citrate | 1,5% |
| Cocamide MEA | 1,0% |
| Sodium Xylenesulfonate | 1,0% |
| Citric Acid | ad pH 5,0 |
| Sodium Benzoate | 0,7% |
| Sodium Chloride | 0,8% |
| Tetrasodium EDTA | 0,3% |
| Polyquaternium-6 | 0,5% |
| Honey (Mel) | 0,5% |
| Prunus Armeniaca (Apricot) Fruit Extract | 0,5% |
| Methylchloroisothiazolinone | 0,1% |
| Methylisothiazolinone | 0,1% |
| Parfum, Dyes | q.s. |

### Formulierung 5: Body Wash

| | |
|---|---|
| Water | ad 100% |
| Sodium Laureth Sulfate | 4,0% |
| Sodium Lauryl Sulfate | 4,0% |
| Sodium Chloride | 3,3% |
| Cocamidopropyl Betaine | 3,0% |
| Rhamnolipid | 3,0% |
| Sodium Benzoate | 1,5% |
| Citric Acid | ad pH 5,5 |
| Disodium EDTA | 0,5% |
| Zea Mays (Corn) Silk Extract | 0,5% |
| Methylchloroisothiazolinone | 0,1% |
| Methylisothiazolinone | 0,1% |
| Potassium Sorbate | 0,2% |
| Parfum, Dyes | q.s. |

### Formulierung 6: Shampoo

| | |
|---|---|
| Aqua | ad 100% |
| Sodium Laureth Sulfate | 9,0% |
| Cocamidopropyl Betaine | 3,0% |
| Rhamnolipid | 3,0% |
| Glycerin | 2,5% |
| Sodium Chloride | 1,7% |
| Glycol Distearate | 1,5% |
| Panthenol | 0,2% |
| Propylene Glycol | 0,3% |
| Hydroxypropyl Guar Hydroxypropyltrimonium Chloride | 0,3% |
| Sodium Benzoate | 0,7% |
| PEG-55 Propylene Glycol Oleate | 0,5% |
| Citric Acid | ad pH 5,5 |
| Hydrolyzed Wheat Protein | 0,3% |
| Argania Spinosa Kernel Oil | 0,1% |
| Laureth-4 | 0,5% |
| Potassium Sorbate | 0,2% |
| Parfum, Dyes | q.s. |

### Formulierung 7: Cool Down Shower Gel

| | |
|---|---|
| Aqua | ad 100% |
| Sodium Laureth Sulfate | 8,0% |
| Cocamidopropyl Betaine | 3,0% |
| Rhamnolipid | 3,0% |
| Glycerin | 2,7% |
| Acrylates Copolymer | 2,5% |
| Sodium Chloride | 2,2% |
| Phenoxyethanol | 1,5% |
| Taurine | 1,5% |
| Benzyl Alcohol | 1,0% |
| Sodium Hydroxide | 0,5% |
| PEG-40 Hydrogenated Castor Oil | 0,7% |
| Citric Acid | ad pH 5,0 |
| Mannitol | 0,7% |
| Benzophenone-4 | 0,3% |
| Ethylhexyl Salicylate | 0,5% |
| Cellulose | 0,5% |
| Linalool | 0,1% |
| Butyl Methoxydibenzoylmethane | 0,2% |
| Hydroxypropyl Methylcellulose | 0,2% |
| Dyes | q.s. |

### Formulierung 8: Gentle Liquid Soap

| | |
|---|---|
| Aqua | ad 100% |
| Sodium Laureth Sulfate | 8,5% |
| Cocamidopropyl Betaine | 3,0% |
| Rhamnolipid | 2,5% |
| PEG-7 Glyceryl Cocoate | 2,5% |
| Cocamide DEA | 2,5% |
| Glycerin | 2,0% |
| Sorbitan Sesquicaprylate | 1,5% |
| C12-15 Pareth-12 | 1,2% |
| Sodium Benzoate | 1,0% |
| Potassium Sorbate | 1,0% |
| Citric Acid | ad pH 5,5 |
| PEG-200 Hydrogenated Glyceryl Palmate | 1,0% |
| Sodium Lactate | 1,0% |
| Sodium Chloride | 0,7% |
| Tetrasodium Glutamate Diacetate | 0,2% |
| Linalool | 0,1% |
| Limonene | 0,1% |
| Citronellol | 0,1% |
| Dyes | q.s. |

### Formulierung 9: Shower Gel and Shampoo

| | |
|---|---|
| Aqua | ad 100% |
| Coco Glucoside | 7,0% |
| Sodium Coco Sulfate | 3,0% |
| Cocamidopropyl Hydroxysultaine | 2,5% |
| Rhamnolipid | 2,0% |
| Glyceryl Oleate | 1,8% |
| Inulin | 1,5% |
| Lecithin | 1,5% |
| Polyquaternium-39 | 1,5% |
| Citric Acid | ad pH 5,0 |
| Styrene/Acrylates Copolymer | 1,0% |
| Sodium Chloride | 1,0% |
| Denatonium Benzoate | 0,5% |
| Hydrogenated Palm Glycerides Citrate | 0,7% |
| Tocopherol | 0,3% |
| Sodium Benzoate | 0,7% |
| Phenoxyethanol | 0,3% |
| Parfum, Dyes | q.s. |

### Formulierung 10: Moisturising Shampoo

| | |
|---|---|
| Aqua | ad 100% |
| Sodium Laureth Sulfate | 8,0% |
| Cocamidopropyl Betaine | 3,0% |
| Sodium Chloride | 2,2% |
| Rhamnolipid | 2,0% |
| Hydrolyzed Keratin | 2,0% |
| Water Lily (Nymphaea Odorata) Root Extract | 1,8% |
| Aloe Barbadensis Leaf Juice | 1,5% |
| Glyceryl Glucoside | 1,0% |
| Panthenol | 1,0% |
| Polyquaternium-10 | 0,2% |
| Guar Hydroxypropyltrimonium Chloride | 0,3% |
| PEG-40 Hydrogenated Castor Oil | 0,7% |
| Glycerin | 0,5% |
| Citric Acid | ad pH 5,0 |
| Sodium Benzoate | 1,2% |
| Propylene Glycol | 1,0% |
| Citronellol | 0,1% |
| Limonene | 0,1% |
| alpha-Isomethyl lonone | 0,1% |
| Benzyl Alcohol | 0,2% |
| Dyes | q.s. |

### Formulierung 11: Shower, Shampoo and Shave

| | |
|---|---|
| Aqua | ad 100% |
| Cocamidopropyl Betaine | 5,0% |
| Sodium Laureth Sulfate | 4,0% |
| PEG-7 Glyceryl Cocoate | 4,0% |
| Sodium Chloride | 3,5% |
| Rhamnolipid | 2,0% |
| PEG-3 Distearate | 3,0% |
| Glycerin | 2,5% |
| Glyceryl Glucoside | 1,2% |
| Helianthus Annuus (Sunflower) Seed Oil | 1,0% |
| Persea Gratissima (Avocado) Oil | 1,0% |
| Polyquaternium-7 | 1,0% |
| PEG-90m | 1,0% |
| PEG-40 Hydrogenated Castor Oil | 1,0% |
| Citric Acid | ad pH 5,0 |
| Benzophenone-4 | 0,5% |
| PEG-200 Hydrogenated Glyceryl Palmate | 0,5% |
| Silica | 0,7% |
| Sodium Benzoate | 0,6% |
| Methylparaben | 0,4% |
| Propylparaben | 0,4% |
| Limonene | 0,1% |
| Butylphenyl Methylpropional | 0,1% |
| Benzyl Alcohol | 0,1% |
| Dyes | q.s. |

### Formulierung 12: Soap

| | |
|---|---|
| Aqua | ad 100% |
| Cocamidopropyl Betaine | 5,0% |
| Sodium Laureth Sulfate | 3,0% |
| Rhamnolipid | 4,0% |
| Glycerin | 2,7% |
| PEG-7 Glyceryl Cocoate | 2,5% |
| Simmondsia Chinensis (Jojoba) Seed Oil | 2,5% |
| Glyceryl Glucoside | 2,3% |
| Mel | 2,0% |
| Sodium Chloride | 2,1% |
| Citric Acid | ad pH 5,0 |
| PEG-40 Hydrogenated Castor Oil | 1,8% |
| PEG-120 Methyl Glucose Dioleate | 1,7% |
| Trisodium EDTA | 1,0% |
| Glycol Distearate | 1,0% |
| Polyquaternium-10 | 0,3% |
| Laureth-10 | 0,7% |
| Benzophenone-4 | 0,5% |
| Cocamide MEA | 0,8% |
| Maltodextrin | 0,5% |
| Sodium Benzoate | 0,7% |
| Sodium Salicylate | 0,6% |
| Benzoic Acid | 0,4% |
| Linalool | 0,1% |
| Parfum, Dyes | q.s. |

### Formulierung 13: Shower Gel

| | |
|---|---|
| Aqua | ad 100% |
| Cocamidopropyl Betaine | 5,0% |
| Sodium Myreth Sulfate | 4,5% |
| Sodium Laureth Sulfate | 4,0% |
| Rhamnolipid | 3,5% |
| PEG-7 Glyceryl Cocoate | 3,2% |
| Glycerin | 3,0% |
| Glyceryl Glucoside | 2,5% |
| Bisabolol | 2,0% |
| Chamomilla Recutita (Matricaria) Flower Extract | 2,2% |
| Sodium Chloride | 2,2% |
| PEG-40 Hydrogenated Castor Oil | 2,0% |
| Citric Acid | ad pH 5,0 |
| Polyquaternium-7 | 1,2% |
| PEG-90 Glyceryl Isostearate | 1,2% |
| PEG-200 Hydrogenated Glyceryl Palmate | 1,2% |
| Benzophenone-4 | 1,0% |
| Laureth-2 | 0,7% |
| BHT | 0,2% |
| Sodium Benzoate | 0,2% |
| Methylparaben | 0,2% |
| Benzyl Alcohol | 0,2% |
| Citronellol | 0,1% |
| Limonene | 0,1% |
| Dyes | q.s. |

### Formulierung 14: Shower Gel

| | |
|---|---|
| Aqua | ad 100% |
| Sodium Laureth Sulfate | 8,0% |
| Cocamidopropyl Betaine | 3,0% |
| Acrylates Copolymer | 3,0% |
| PEG-7 Glyceryl Cocoate | 2,7% |
| Rhamnolipid | 2,5% |
| Helianthus Annuus Seed Oil | 1,5% |
| Glycerin | 1,5% |
| Glyceryl Glucoside | 1,5% |
| Sodium Chloride | 1,0% |
| PEG-40 Hydrogenated Castor Oil | 1,0% |
| Disodium Cocoyl Glutamate | 1,0% |
| Trisodium EDTA | 1,0% |
| Propylene Glycol | 1,0% |
| Microcrystalline Cellulose | 0,9% |
| Benzophenone-4 | 0,5% |
| Sodium Sulfate | 0,7% |
| Phenoxyethanol | 0,7% |
| Propylparaben | 0,1% |
| Methylparaben | 0,1% |
| Benzyl Alcohol | 0,2% |
| Parfum, Dyes | q.s. |

### Formulierung 15: Shampoo

| | |
|---|---|
| Aqua | ad 100% |
| Decyl Glucoside | 6,0% |
| Sodium Myreth Sulfate | 5,5% |
| Rhamnolipid | 3,0% |
| PEG-200 Hydrogenated Glyceryl Palmate | 2,5% |
| PEG-80 Sorbitan Laurate | 2,0% |
| PEG-90 Glycol Isostearate | 2,0% |
| Disodium PEG-5 Laurylcitrate Sulfosuccinate | 1,5% |
| Polyquaternium-10 | 1,2% |
| Hydrolyzed Silk | 1,2% |
| Laureth-2 | 1,2% |
| Citric Acid | ad pH 5,5 |
| Sodium Laureth Sulfate | 1,0% |
| PEG-40 Hydrogenated Castor Oil | 0,9% |
| Sodium Benzoate | 0,5% |
| Parfum, Dyes | q.s. |

### Formulierung 16: Creme Oil Bath

| | |
|---|---|
| Aqua | ad 100% |
| Sodium Laureth Sulfate | 5,0% |
| Cocamidopropyl Betaine | 3,0% |
| Decyl Glucoside | 2,8% |
| Rhamnolipid | 2,5% |
| Glycerin | 2,5% |
| Glyceryl Glucoside | 2,0% |
| Diamond Powder | 2,0% |
| Helianthus Annuus (Sunflower) Seed Oil | 1,7% |
| Sodium Chloride | 1,5% |
| Glycol Distearate | 1,5% |
| PEG-40 Hydrogenated Castor Oil | 1,5% |
| Citric Acid | ad pH 5,5 |
| PEG-90 Glyceryl Isostearate | 1,2% |
| Laureth-4 | 1,0% |
| PEG-200 Hydrogenated Glyceryl Palmate | 1,0% |
| PEG-7 Glyceryl Cocoate | 1,0% |
| Benzophenone-4 | 0,9% |
| Laureth-2 | 0,7% |
| Sodium Benzoate | 0,7% |
| Butylphenyl Methylpropional | 0,1% |
| Linalool | 0,1% |
| Limonene | 0,1% |
| alpha-Isomethyl lonone | 0,1% |
| Dyes | q.s. |

### Formulierung 17: Cleansing Oil Shampoo

| | |
|---|---|
| Aqua | ad 100% |
| Sodium Laureth Sulfate | 7,0% |
| MIPA-Laureth Sulfate | 4,0% |
| Sodium Chloride | 3,2% |
| Cocamidopropyl Betaine | 3,0% |
| Rhamnolipid | 3,0% |
| Glycerin | 2,5% |
| PEG-18 Castor Oil Dioleate | 2,0% |
| Propylene Glycol | 2,0% |
| PEG-55 Propylene Glycol Oleate | 1,7% |
| Laureth-5 Carboxylic Acid | 1,0% |
| Persea Gratissima (Avocado) Oil | 1,0% |
| PPG-5-Ceteth-20 | 1,0% |
| Sodium Benzoate | 0,7% |
| Laureth-2 | 0,5% |
| Salicylic Acid | 0,3% |
| Linalool | 0,2% |
| alpha-Isomethyl lonone | 0,1% |
| Limonene | 0,1% |
| Zea Mays (Corn) Germ Oil | 0,2% |
| Argania Spinosa Oil Kernel Oil | 0,1% |
| Camellia Oleifera Seed Oil | 0,1% |
| Sodium Hydroxide | 0,3% |
| Citric Acid | ad pH 5,0 |
| Parfum, Dyes | q.s. |

**Formulierung 18: Shine Shampoo**

| | |
|---|---|
| Aqua | ad 100% |
| Ammonium Lauryl Sulfate | 5,5% |
| Rhamnolipid | 3,0% |
| Cocamidopropyl Betaine | 3,0% |
| Sodium Chloride | 2,3% |
| Cocamide MEA | 2,0% |
| Nacre Powder | 1,0% |
| Sodium Benzoate | 0,7% |
| Hydroxypropyl Guar Hydroxypropyltrimonium Chloride | 0,7% |
| Hydroxypropyltrimonium Hydrolyzed Wheat Protein | 0,7% |
| Sodium Hydroxide | 0,2% |
| Salicylic Acid | 0,5% |
| Limonene | 0,1% |
| Benzoic Acid | 0,5% |
| Linalool | 0,3% |
| Benzyl Alcohol | 0,5% |
| Tin Oxide | 0,2% |
| alpha-Isomethyl lonone | 0,1% |
| Acrylates Copolymer | 0,1% |
| Citric Acid | ad pH 5,5 |
| Citronellol | 0,1% |
| Hexyl Cinnamal | 0,1% |
| Dyes | q.s. |

### Formulierung 19: Shampoo

| | |
|---|---|
| Aqua | ad 100% |
| Sodium Cocoyl Isethionate | 3,5% |
| Sodium Lauryl Sulfoacetate | 3,5% |
| Disodium Laureth Sulfosuccinate | 3,5% |
| Cocamidopropyl Betaine | 3,0% |
| Rhamnolipid | 3,0% |
| Sodium Lauroyl Sarcosinate | 1,0% |
| Glycol Distearate | 1,0% |
| C11-15 Pareth-7 | 1,0% |
| C12-13 Pareth-23 | 1,0% |
| C12-13 Pareth-3 | 1,0% |
| Decyl Glucoside | 1,0% |
| Tocopherol | 1,0% |
| Hydrogenated Coconut Acid | 1,0% |
| Sodium Chloride | 0,8% |
| Sodium Isethionate | 0,7% |
| Sodium Benzoate | 0,7% |
| Sodium Hydroxide | 0,3% |
| Phenoxyethanol | 0,6% |
| PPG-5 Ceteth-20 | 0,5% |
| PEG-55 Propylene Glycol Oleate | 0,5% |
| Trideceth-12 | 0,5% |
| Polyquaternium-7 | 0,5% |
| Polyquaternium-10 | 0,3% |
| Limonene | 0,1% |
| Benzoic Acid | 0,3% |
| Benzophenone-4 | 0,3% |
| Benzyl Salicylate | 0,2% |
| Linalool | 0,6% |
| Benzyl Alcohol | 0,6% |
| Amodimethicone | 0,5% |
| Propylene Glycol | 0,5% |
| Carbomer | 0,7% |
| Rosmarinus Officinalis (Rosemary) Leaf Oil | 0,7% |
| Methylparaben | 0,3% |
| Methylchloroisothiazolinone | 0,1% |
| Methylisothiazolinone | 0,1% |
| Citric Acid | ad pH 5,0 |
| Laureth-9 | 0,5% |
| Divinyldimethicone/Dimethicone Copolymer | 0,2% |
| Glycerin | 0,2% |
| Parfum, Dyes | q.s. |

### Formulierung 20: Reinforcing Shampoo

| | |
|---|---|
| Aqua | ad 100% |
| Sodium Laureth Sulfate | 6,0% |
| Dimethicone | 3,0% |
| Sodium Chloride | 3,0% |
| Coco-Betaine | 3,0% |
| Rhamnolipid | 2,0% |
| Glycol Distearate | 1,0% |
| Guar Hydroxypropyltrimonium Chloride | 0,3% |
| Cocamide MIPA | 0,5% |
| Sodium Benzoate | 0,7% |
| Hydroxypropyltrimonium Hydrolyzed Wheat Protein | 0,3% |
| Sodium Cocoate | 0,7% |
| Sodium Hydroxide | 0,6% |
| Arginine | 0,7% |
| Salicylic Acid | 0,5% |
| Limonene | 0,2% |
| Linalool | 0,2% |
| 2-Oleamido-1,3-Octadecanediol | 0,1% |
| Carbomer | 0,5% |
| Methyl Cocoate | 0,5% |
| Citric Acid | ad pH 5,0 |
| Hexylene Glycol | 0,2% |
| Hexyl Cinnamal | 0,1% |
| Glyceryl Linoleate | 0,1% |
| Glyceryl Oleate | 0,2% |
| Glyceryl Linolenate | 0,2% |
| Dyes | q.s. |

### Formulierung 21: Shampoo

| | |
|---|---|
| Aqua | ad 100% |
| Sodium Laureth Sulfate | 9,0% |
| Dimethicone | 3,5% |
| Sodium Chloride | 3,2% |
| Coco-Betaine | 3,0% |
| Glycol Distearate | 3,0% |
| Rhamnolipid | 2,0% |
| Guar Hydroxypropyltrimonium Chloride | 0,3% |
| Niacinamide | 0,1% |
| Cocamide MIPA | 2,0% |
| Saccharum Officinarum (Sugar Cane) Extract | 2,0% |
| Sodium Benzoate | 1,0% |
| Sodium Cocoate | 1,5% |
| Sodium Hydroxide | 0,6% |
| Salicylic Acid | 0,5% |
| Camellia Sinensis Extract/Camellia Sinensis Leaf Extract | 0,5% |
| Linalool | 0,2% |
| Benzyl Salicylate | 0,2% |
| Pyrus Malus (Apple) Fruit Extract | 0,3% |
| Carbomer | 0,5% |
| Pyridoxine HCl | 0,3% |
| Persea Gratissima (Avocado) Oil | 0,2% |
| Citric Acid | ad pH 5,5 |
| Butylphenyl Methylpropional | 0,1% |
| Methyl Cocoate | 0,3% |
| Hexylene Glycol | 0,1% |
| Hexyl Cinnamal | 0,1% |
| Dyes | q.s. |

### Formulierung 22: Fortifying Shampoo

| | |
|---|---|
| Aqua | ad 100% |
| Ammonium Lauryl Sulfate | 6,0% |
| Cocamidopropyl Betaine | 3,0% |
| Sodium Chloride | 2,7% |
| Rhamnolipid | 2,0% |
| Niacinamide | 0,1% |
| Saccharum Officinarum (Sugar Cane) Extract | 0,9% |
| Cocos Nucifera (Coconut) Oil | 0,9% |
| Sodium Benzoate | 0,9% |
| Hydroxypropyl Guar Hydroxypropyltrimonium Chloride | 0,1% |
| Sodium Hydroxide | ad pH |
| Salicylic Acid | 0,4% |
| Limonene | 0,1% |
| Benzoic Acid | 0,4% |
| Linalool | 0,3% |
| Cinnamyl Alcohol | 0,2% |
| Pyrus Malus (Apple) Fruit Extract | 0,5% |
| Pyridoxine HCl | 0,2% |
| Bisabolol | 0,2% |
| Citric Acid | ad pH 5,0 |
| Citronellol | 0,1% |
| Hexylene Glycol | 0,2% |
| Dyes | q.s. |

### Formulierung 23: 2 in 1 Shampoo

| | |
|---|---|
| Aqua | ad 100% |
| Sodium Laureth Sulfate | 8,0% |
| PEG-200 Hydrogenated Glyceryl Palmate | 3,0% |
| Rhamnolipid | 3,0% |
| Disodium Cocoamphodiacetate | 3,0% |
| Polysorbate 20 | 2,5% |
| Glycerin | 2,5% |
| Sodium Chloride | 2,0% |
| PEG-7 Glyceryl Cocoate | 2,0% |
| Sodium Benzoate | 1,0% |
| Sodium Laureth-8 Sulfate | 1,0% |
| Sodium Oleth Sulfate | 1,0% |
| Sodium Hydroxide | 0,3% |
| PPG-5 Ceteth-20 | 1,0% |
| PEG-55 Propylene Glycol Oleate | 0,5% |
| Polyquaternium-10 | 0,2% |
| Salicylic Acid | 0,3% |
| Maltodextrin | 0,2% |
| Propylene Glycol | 0,2% |
| Disodium Ricinoleamido MEA-Sulfosuccinate | 0,1% |
| Citric Acid | ad pH 5,5 |
| Prunus Armeniaca (Apricot) Kernel Oil | 0,1% |
| Parfum, Dyes | q.s. |

### Formulierung 24: Body Wash

| | |
|---|---|
| Aqua | ad 100% |
| Sodium Hydroxypropyl Starch Phosphate | 6,0% |
| Sodium Laureth Sulfate | 5,5% |
| Cocamidopropyl Betaine | 3,0% |
| Rhamnolipid | 3,0% |
| Lauric Acid | 2,5% |
| Petrolatum | 2,0% |
| Sodium Cocoyl Glycinate | 2,0% |
| Glycerin | 1,7% |
| Sodium Lauroyl Isethionate | 1,5% |
| Glycine Soja (Soybean) Oil | 1,0% |
| Helianthus Annuus (Sunflower) Oil | 1,0% |
| Sodium Chloride | 1,0% |
| Stearic Acid | 0,7% |
| Guar Hydroxypropyltrimonium Chloride | 0,3% |
| DMDM Hydantoin | 0,1% |
| Tallow Acid | 0,2% |
| Palmitic Acid | 0,1% |
| Sodium Isethionate | 0,2% |
| BHT | 0,1% |
| Tetrasodium EDTA | 0,2% |
| Methylisothiazolinone | 0,3% |
| lodopropynyl Butylcarbamate | 0,1% |
| Titanium Dioxide | 0,1% |
| Parfum, Dyes | q.s. |

### Formulierung 25: Shower Cream

| | |
|---|---|
| Aqua | ad 100% |
| Sodium Laureth Sulfate | 7,0% |
| Cocamidopropyl Betaine | 3,0% |
| Rhamnolipid | 2,0% |
| Sodium Chloride | 1,9% |
| Polyquaternium-7 | 1,5% |
| Cocamide MEA | 1,5% |
| PEG-7 Glyceryl Cocoate | 1,0% |
| Laureth-10 | 1,0% |
| Glycol Distearate | 0,8% |
| Citric Acid | ad pH 5,0 |
| Linalool | 0,1% |
| Limonene | 0,1% |
| Benzyl Salicylate | 0,1% |
| Butylphenyl Methylpropional | 0,1% |
| alpha-Isomethyl lonone | 0,1% |
| Hexyl Cinnamal | 0,1% |
| Citronellol | 0,1% |
| Sodium Benzoate | 0,7% |
| Sodium Salicylate | 0,1% |
| Dyes | q.s. |

### Formulierung 26: 2 in 1 Body Wash

| | |
|---|---|
| Aqua | ad 100% |
| Sodium Laureth Sulfate | 8,5% |
| Glycerin | 2,5% |
| Sodium Cocoyl Isethionate | 2,5% |
| Ammonium Lauryl Sulfate | 2,5% |
| Lauryl Betaine | 2,5% |
| Lauryl Glucoside | 1,5% |
| Sodium Chloride | 1,0% |
| Rhamnolipid | 1,0% |
| Polyquaternium-39 | 0,5% |
| Polyquaternium-7 | 0,5% |
| Panthenol | 0,1% |
| Citric Acid | ad pH 5,5 |
| Sodium Citrate | 0,2% |
| Propylene Glycol | 0,3% |
| PEG-55 Propylene Glycol Oleate | 0,7% |
| Styrene/Acrylates Copolymer | 0,2% |
| Phenoxyethanol | 0,7% |
| Sodium Benzoate | 0,3% |
| Tetrasodium EDTA | 0,1% |
| Parfum, Dyes | q.s. |

### Formulierung 27: 2 in 1 Bubble Bath and Wash

| | |
|---|---|
| Aqua | ad 100% |
| Cocamidopropyl Betaine | 4,0% |
| Glycerin | 4,0% |
| Sodium Lauroamphodiacetate | 2,0% |
| Sodium Chloride | 2,0% |
| Coco Glucoside | 1,5% |
| Hydroxypropyl Starch Phosphate | 1,0% |
| Disodium Lauroamphodiacetate | 1,0% |
| Rhamnolipid | 1,0% |
| Lauric Acid | 1,0% |
| Acrylates/C10-30 Alkyl Acrylate Crosspolymer | 1,0% |
| Polyquaternium-7 | 1,0% |
| Styrene/Acrylates Copolymer | 0,5% |
| Citric Acid | ad pH 5,5 |
| Sodium Hydroxide | 0,3% |
| Sodium Benzoate | 0,2% |
| Benzoic Acid | 0,1% |
| Parfum, Dyes | q.s. |

### Formulierung 28: Body and Face Wash

| | |
|---|---|
| Aqua | ad 100% |
| Sodium Laureth Sulfate | 7,0% |
| Petrolatum | 3,0% |
| Acrylates Copolymer | 2,5% |
| Cocamide MEA | 2,5% |
| Rhamnolipid | 2,0% |
| PPG-9 | 2,0% |
| Menthol | 0,2% |
| DMDM Hydantoin | 0,1% |
| Tetrasodium EDTA | 0,2% |
| Methylisothiazolinone | 0,1% |
| BHT | 0,1% |
| Parfum, Dyes | q.s. |

### Formulierung 29: Anti-Aging Body Wash

| | |
|---|---|
| Aqua | ad 100% |
| Petrolatum | 5,0% |
| Mineral (Paraffinum Liquidum) Oil | 4,0% |
| Sodium Trideceth Sulfate | 3,5% |
| Sodium Lauryl Sulfate | 3,0% |
| Sodium Lauroamphoacetate | 3,0% |
| Sodium Chloride | 2,3% |
| Rhamnolipid | 2,0% |
| Trideceth-3 | 1,0% |
| Simmondsia Chinensis (Jojoba) Butter | 0,7% |
| Niacinamide | 0,1% |
| Panthenol | 0,3% |
| Soluble Collagen | 0,5% |
| Citric Acid | ad pH 5,5 |
| Disodium EDTA | 0,3% |
| Guar Hydroxypropyltrimonium Chloride | 0,3% |
| Methylchloroisothiazolinone | 0,1% |
| Methylisothiazolinone | 0,1% |
| PEG-90m | 0,7% |
| Sodium Benzoate | 0,3% |
| Sodium Hydroxide | 0,1% |
| Xanthan Gum | 0,5% |
| Parfum, Dyes | q.s. |

### Formulierung 30: Moisturising Shower Cream

| | |
|---|---|
| Aqua | ad 100% |
| Sodium Laureth Sulfate | 8,5% |
| Cocamidopropyl Betaine | 3,0% |
| Glycerin | 2,5% |
| Rhamnolipid | 2,0% |
| Sodium Chloride | 1,7% |
| Cocamide MEA | 1,5% |
| Styrene/Acrylates Copolymer | 1,5% |
| Sodium Salicylate | 1,0% |
| Sodium Benzoate | 1,0% |
| Polyquaternium-7 | 1,0% |
| Citric Acid | ad pH 5,0 |
| Tetrasodium EDTA | 1,0% |
| Glycol Distearate | 0,9% |
| Laureth-4 | 0,5% |
| Magnolia Officinalis Flower Extract | 0,2% |
| Butyrospermum Parkii Butter Extract | 0,2% |
| Prunus Amygdalus Dulcis (Sweet Almond) Extract | 0,1% |
| alpha-Isomethyl lonone | 0,1% |
| Benzyl Benzoate | 0,1% |
| Cinnamyl Alcohol | 0,1% |
| Hexyl Cinnamal | 0,1% |
| Parfum, Dyes | q.s. |

### Formulierung 31: Bath

| | |
|---|---|
| Aqua | ad 100% |
| PEG-80 Sorbitan Laurate | 4,0% |
| Cocamidopropyl Betaine | 4,0% |
| Sodium Laureth Sulfate | 4,0% |
| Rhamnolipid | 2,0% |
| Polysorbate 20 | 2,0% |
| Sodium Chloride | 1,5% |
| Citric Acid | ad pH 5,0 |
| Sodium Lauroamphoacetate | 1,0% |
| PEG-150 Distearate | 1,0% |
| Disodium Lauroamphodiacetate | 1,0% |
| Sodium Benzoate | 0,5% |
| Parfum, Dyes | q.s. |

### Formulierung 32: Shower Gel

| | |
|---|---|
| Aqua | ad 100% |
| Sodium Laureth Sulfate | 9,0% |
| Cocamidopropyl Betaine | 3,0% |
| Rhamnolipid | 3,0% |
| Glycerin | 3,0% |
| Sodium Chloride | 2,8% |
| PEG-7 Glyceryl Cocoate | 2,5% |
| PEG-40 Hydrogenated Castor Oil | 2,5% |
| Citric Acid | ad pH 5,5 |
| Sodium Benzoate | 1,0% |
| Potassium Sorbate | 0,7% |
| Alcohol | 0,5% |
| Citronellol | 0,2% |
| Benzyl Alcohol | 0,7% |
| Limonene | 0,1% |
| Linalool | 0,2% |
| Geraniol | 0,2% |
| Hexyl Cinnamal | 0,1% |
| Ethylhexyl Salicylate | 0,1% |
| Octocrylene | 0,3% |
| Butyl Methoxydibenzoylmethane | 0,2% |
| Dyes | q.s. |

### Formulierung 33: Shower Gel

| | |
|---|---|
| Aqua | ad 100% |
| Alcohol | 6,0% |
| Coco Glucoside | 6,0% |
| Glycerin | 3,0% |
| Rhamnolipid | 3,0% |
| Xanthan Gum | 1,5% |
| Disodium Cocoyl Glutamate | 2,0% |
| Citric Acid | ad pH 5,5 |
| Citrus Aurantifolia (Lime) Oil | 0,5% |
| Sodium Cocoyl Glutamate | 0,7% |
| Glyceryl Oleate | 0,3% |
| Sodium Lauroyl Lactylate | 0,8% |
| Limonene | 0,1% |
| Linalool | 0,1% |
| Dyes | q.s. |

### Formulierung 34: Hair and Body Shampoo

| | |
|---|---|
| Aqua | ad 100% |
| Sodium Laureth Sulfate | 10,0% |
| Sodium Chloride | 4,0% |
| Glycerin | 3,5% |
| Coco-Betaine | 2,0% |
| Rhamnolipid | 2,0% |
| Niacinamide | 0,1% |
| Saccharum Officinarum (Sugar Cane) Extract | 1,0% |
| Sodium Benzoate | 1,0% |
| Sodium Hydroxide | 0,3% |
| PPG-5-Ceteth-20 | 1,0% |
| Polyquaternium-10 | 0,7% |
| Salicylic Acid | 0,5% |
| Limonene | 0,1% |
| Linalool | 0,2% |
| Benzyl Salicylate | 0,1% |
| Pyrus Malus (Apple Fruit) Extract | 0,3% |
| Pyridoxine HCl | 0,2% |
| Citric Acid | ad pH 5,0 |
| Taurine | 0,1% |
| Dyes | q.s. |

### Formulierung 35: Shampoo

| | |
|---|---|
| Aqua | ad 100% |
| Sodium Laureth Sulfate | 9,0% |
| Cocamidopropyl Betaine | 2,5% |
| Rhamnolipid | 2,5% |
| Sodium Chloride | 2,5% |
| Camellia Sinensis Leaf Extract | 2,0% |
| Guar Hydroxypropyltrimonium Chloride | 0,3% |
| Maltodextrin | 1,2% |
| Disodium EDTA | 1,0% |
| PPG-12 | 0,7% |
| Citric Acid | ad pH 5,0 |
| Sodium Hydroxide | 0,3% |
| Sodium Benzoate | 0,7% |
| Butylphenyl Methylpropional | 0,2% |
| Geraniol | 0,2% |
| Hexyl Cinnamal | 0,1% |
| Limonene | 0,1% |
| Linalool | 0,1% |
| Dyes | q.s. |

### Formulierung 36: Brunette Shampoo

| | |
|---|---|
| Aqua | ad 100% |
| Sodium Laureth Sulfate | 8,5% |
| Cocamidopropyl Betaine | 4,0% |
| Sodium Chloride | 3,5% |
| Rhamnolipid | 2,0% |
| Selaginella Lepidophylla Aerial Extract | 2,0% |
| Gluconolactone | 1,8% |
| Glycerin | 1,8% |
| Dimethiconol | 1,0% |
| Guar Hydroxypropyltrimonium Chloride | 0,5% |
| TEA-Dodecylbenzenesulfonate | 1,0% |
| Maltodextrin | 1,0% |
| Disodium EDTA | 0,7% |
| Carbomer | 0,9% |
| PPG-12 | 0,7% |
| Citric Acid | ad pH 5,5 |
| Sodium Hydroxide | 0,9% |
| TEA-Sulfate | 0,7% |
| Triethanolamine | 0,5% |
| DMDM Hydantoin | 0,1% |
| Sodium Benzoate | 0,7% |
| Methylchloroisothiazolinone | 0,1% |
| Methylisothiazolinone | 0,1% |
| alpha-Isomethyl lonone | 0,1% |
| Butylphenyl Methylpropional | 0,2% |
| Hexyl Cinnamal | 0,1% |
| Limonene | 0,1% |
| Linalool | 0,1% |
| Dyes | q.s. |

### Formulierung 37: Shower Gel for Men

| | |
|---|---|
| Aqua | ad 100% |
| Sodium Laureth Sulfate | 9,5% |
| Cocamidopropyl Betaine | 3,0% |
| Cocamide MEA | 3,0% |
| Rhamnolipid | 3,0% |
| Glycerin | 2,5% |
| PPG-12 | 2,0% |
| Sodium Chloride | 2,0% |
| Citric Acid | ad pH 5,5 |
| Sodium Benzoate | 1,0% |
| Phenoxyethanol | 0,7% |
| Benzyl Alcohol | 0,7% |
| Butylphenyl Methylpropional | 0,1% |
| Citronellol | 0,1% |
| Geraniol | 0,2% |
| Hexyl Cinnamal | 0,1% |
| Limonene | 0,1% |
| Linalool | 0,1% |
| Dyes | q.s. |

### Formulierung 38: Caring Liquid Soap

| | |
|---|---|
| Aqua | ad 100% |
| Aloe Barbadensis Leaf Juice | 5,0% |
| Ammonium Lauryl Sulfate | 5,0% |
| Coco Glucoside | 4,0% |
| Cocamidopropyl Betaine | 4,0% |
| Sodium Cocoamphoacetate | 1,5% |
| Rhamnolipid | 1,5% |
| Glycerin | 1,5% |
| Sodium Chloride | 1,5% |
| Citric Acid | ad pH 5,5 |
| Sodium Benzoate | 1,0% |
| Sodium Salicylate | 0,5% |
| Methylisothiazolinone | 0,3% |
| Linalool | 0,1% |
| Butylphenyl Methylpropional | 0,2% |
| Limonene | 0,1% |
| Dyes | q.s. |

### Formulierung 39: Shower Cream

| | |
|---|---|
| Aqua | ad 100% |
| Ammonium Lauryl Sulfate | 6,0% |
| Coco Glucoside | 3,5% |
| Cocamidopropyl Betaine | 2,0% |
| Sodium Cocoamphoacetate | 2,0% |
| Rhamnolipid | 1,5% |
| Argania Spinosa Kernel Oil | 1,0% |
| Butyrospermum Parkii Butter | 1,0% |
| Glycerin | 1,0% |
| Polyquaternium-7 | 1,0% |
| Styrene/Acrylates Copolymer | 1,0% |
| Sodium Chloride | 1,0% |
| Citric Acid | ad pH 5,0 |
| Sodium Benzoate | 0,3% |
| Sodium Salicylate | 0,1% |
| Methylparaben | 0,3% |
| Linalool | 0,1% |
| Dyes | q.s. |

### Formulierung 40: Shampoo

| | |
|---|---|
| Aqua | ad 100% |
| Sodium Laureth Sulfate | 5,0% |
| Disodium Cocoamphodiacetate | 2,0% |
| Rhamnolipid | 2,0% |
| Laureth-2 | 0,3% |
| Panthenol | 0,2% |
| Niacinamide | 0,1% |
| Sodium Chloride | 0,7% |
| Citric Acid | ad pH 5,0 |
| PEG-7 Glyceryl Cocoate | 0,5% |
| Sodium Benzoate | 0,5% |
| Polyquaternium-10 | 0,7% |
| Salicylic Acid | 0,5% |
| Hexyl Salicylate | 0,2% |
| Benzyl Salicylate | 0,1% |
| Hexyl Cinnamal | 0,1% |
| Linalool | 0,1% |
| Limonene | 0,1% |
| Propylene Glycol | 0,2% |
| Dyes | q.s. |

### Formulierung 41: Intensive Care Oil-Shampoo

| | |
|---|---|
| Aqua | ad 100% |
| Sodium Laureth Sulfate | 8,0% |
| Disodium Cocoamphodiacetate | 3,0% |
| Rhamnolipid | 2,5% |
| Prunus Armeniaca (Apricot) Kernel Oil | 2,5% |
| Hydrolyzed Keratin | 2,5% |
| Panthenol | 2,4% |
| Sodium Chloride | 2,2% |
| Citric Acid | ad pH 5,5 |
| Polyquaternium-10 | 0,5% |
| Sodium Benzoate | 0,5% |
| PEG-7 Glyceryl Cocoate | 0,7% |
| Laureth-2 | 0,4% |
| PEG-40 Hydrogenated Castor Oil | 0,4% |
| Propylene Glycol | 0,8% |
| PEG-55 Propylene Glycol Oleate | 0,7% |
| Hexyl Cinnamal | 0,1% |
| Butylphenyl Methylpropional | 0,2% |
| Benzyl Salicylate | 0,1% |
| Linalool | 0,2% |
| Benzyl Alcohol | 0,5% |
| Limonene | 0,1% |
| Sodium Acetate | 0,2% |
| Dyes | q.s. |

### Formulierung 42: Shampoo

| | |
|---|---|
| Aqua | ad 100% |
| Sodium Laureth Sulfate | 8,5% |
| Disodium Cocoamphodiacetate | 3,5% |
| Sodium Chloride | 3,5% |
| Rhamnolipid | 3,0% |
| Hydrolyzed Collagen | 1,0% |
| Panthenol | 0,4% |
| Cocodimonium Hydroxypropyl Hydrolyzed Keratin | 0,3% |
| Hydrolyzed Keratin | 0,3% |
| Citric Acid | ad pH 5,5 |
| Sodium Benzoate | 0,7% |
| Polyquaternium-10 | 0,2% |
| Hexyl Cinnamal | 0,1% |
| Limonene | 0,1% |
| Linalool | 0,1% |
| Benzyl Salicylate | 0,1% |
| Benzyl Alcohol | 0,2% |
| Propylene Glycol | 0,2% |
| Dyes | q.s. |

### Formulierung 43: Shampoo

| | |
|---|---|
| Aqua | ad 100% |
| Sodium Laureth Sulfate | 8,0% |
| Cocamidopropyl Betaine | 3,0% |
| Rhamnolipid | 3,0% |
| PEG-7 Glyceryl Cocoate | 2,5% |
| Dimethicone | 2,5% |
| Prunus Armeniaca (Apricot) Kernel Oil | 2,5% |
| Panthenol | 0,3% |
| Cocodimonium Hydroxypropyl Hydrolyzed Keratin | 0,7% |
| Hydrolyzed Keratin | 0,7% |
| Sodium Chloride | 0,8% |
| Cocamide MEA | 0,5% |
| Glycol Distearate | 0,2% |
| Sodium Benzoate | 0,7% |
| Citric Acid | ad pH 5,5 |
| Polyquaternium-10 | 0,5% |
| Laureth-4 | 0,4% |
| PEG-40 Hydrogenated Castor Oil | 0,2% |
| Hydrogenated Castor Oil | 0,7% |
| Laureth-7 | 0,5% |
| Hexyl Cinnamal | 0,1% |
| Propylene Glycol | 0,8% |
| Hexyl Salicylate | 0,3% |
| Glycerin | 0,5% |
| Linalool | 0,2% |
| Limonene | 0,1% |
| Dyes | q.s. |

### Formulierung 44: Hair & Body Shampoo & Shower Gel

| | |
|---|---|
| Aqua | ad 100% |
| Sodium Laureth Sulfate | 7,0% |
| Sodium Chloride | 3,8% |
| Cocamidopropyl Betaine | 3,5% |
| Rhamnolipid | 3,0% |
| Panthenol | 0,3% |
| Hydrolyzed Keratin | 0,7% |
| Taurine | 0,5% |
| Simmondsia Chinensis Seed Oil | 0,5% |
| Disodium Cocoamphodiacetate | 0,5% |
| Citric Acid | ad pH 5,0 |
| Sodium Benzoate | 0,7% |
| Cocamide MEA | 0,4% |
| PEG-7 Glyceryl Cocoate | 0,3% |
| Polyquaternium-10 | 0,2% |
| Propylene Glycol | 0,3% |
| PEG-40 Hydrogenated Castor Oil | 0,4% |
| Linalool | 0,2% |
| Geraniol | 0,1% |
| Butylphenyl Methylpropional | 0,1% |
| Dyes | q.s. |

### Formulierung 45: Intensive Cream Shampoo

| | |
|---|---|
| Aqua | ad 100% |
| Sodium Laureth Sulfate | 8,5% |
| Cocamidopropyl Betaine | 3,0% |
| Rhamnolipid | 2,5% |
| Sodium Chloride | 2,5% |
| Panthenol | 0,5% |
| Hydrolyzed Keratin | 0,8% |
| Argania Spinosa Kernel Oil | 0,5% |
| Cocamide MEA | 0,5% |
| Glycol Distearate | 0,5% |
| PEG-7 Glyceryl Cocoate | 0,4% |
| Sodium Benzoate | 0,7% |
| Citric Acid | ad pH 5,0 |
| Laureth-4 | 0,3% |
| PEG-40 Hydrogenated Castor Oil | 0,4% |
| Hydrogenated Castor Oil | 0,4% |
| Styrene/Acrylates Copolymer | 0,5% |
| Dimethicone | 0,8% |
| Polyquaternium-7 | 0,9% |
| PEG-12 Dimethicone | 0,5% |
| Guar Hydroxypropyltrimonium Chloride | 0,3% |
| Benzyl Salicylate | 0,1% |
| Propylene Glycol | 0,8% |
| Glycerin | 0,5% |
| Limonene | 0,1% |
| Linalool | 0,1% |
| PEG-14m | 0,1% |
| Dyes | q.s. |

### Formulierung 46: Shampoo

| | |
|---|---|
| Aqua | ad 100% |
| Sodium Laureth Sulfate | 7,5% |
| Cocamidopropyl Betaine | 3,0% |
| Rhamnolipid | 2,5% |
| Sodium Chloride | 2,0% |
| Sodium Xylenesulfonate | 1,7% |
| Cocamide MEA | 1,5% |
| Sodium Citrate | 1,5% |
| Citric Acid | ad pH 5,0 |
| Dimethiconol | 1,0% |
| Cassia Hydroxypropyltrimonium Chloride | 0,3% |
| Sodium Benzoate | 0,9% |
| Disodium EDTA | 0,7% |
| Panthenol | 0,7% |
| Panthenyl Ethyl Ether | 0,3% |
| Persea Gratissima (Avocado) Oil | 0,2% |
| Methylchloroisothiazolinone | 0,1% |
| Methylisothiazolinone | 0,1% |
| Parfum, Dyes | q.s. |

### Formulierung 47: Caffeine Shampoo

| | |
|---|---|
| Aqua | ad 100% |
| Sodium Laureth Sulfate | 6,0% |
| Laureth-2 | 4,0% |
| Disodium Laureth Sulfosuccinate | 2,5% |
| Sodium Lauroyl Glutamate | 2,5% |
| Rhamnolipid | 2,0% |
| Sodium Chloride | 2,0% |
| Caffeine | 1,5% |
| Panthenol | 0,2% |
| PEG-120 Methyl Glucose Dioleate | 0,5% |
| Hydrolysed Wheat Protein | 0,7% |
| Citric Acid | ad pH 5,5 |
| Sodium Citrate | 0,5% |
| Menthol | 0,7% |
| PEG-40 Hydrogenated Castor Oil | 0,4% |
| Potassium Sorbate | 0,3% |
| Polyquaternium-7 | 0,4% |
| Disodium EDTA | 0,5% |
| Sodium Benzoate | 0,7% |
| Zinc PCA | 0,3% |
| Niacinamide | 0,1% |
| Limonene | 0,1% |
| Tocopherol | 0,1% |
| Phenoxyethanol | 0,5% |
| Methylparaben | 0,3% |
| Propylparaben | 0,3% |
| Dyes | q.s. |

### Formulierung 48: Power Grey Shampoo

| | |
|---|---|
| Aqua | ad 100% |
| Sodium Laureth Sulfate | 6,5% |
| Sodium Cocoamphoacetate | 4,5% |
| PEG-3 Distearate | 3,0% |
| Cocamidopropyl Betaine | 3,0% |
| Rhamnolipid | 2,5% |
| Sodium Chloride | 2,5% |
| Caffeine | 1,5% |
| Panthenol | 0,2% |
| Phenoxyethanol | 0,7% |
| Coco Glucoside | 0,9% |
| Glyceryl Oleate | 0,4% |
| Phosphoric Acid | 0,2% |
| PEG-120 Methyl Glucose Dioleate | 0,9% |
| Menthol | 0,9% |
| Polyquaternium-7 | 0,7% |
| Sodium Hydroxide | 0,3% |
| Citric Acid | ad pH 5,5 |
| Niacinamide | 0,1% |
| Zinc PCA | 0,5% |
| Limonene | 0,1% |
| Sodium Benzoate | 0,2% |
| Methylparaben | 0,3% |
| Propylparaben | 0,3% |
| Dyes | q.s. |

### Formulierung 49: Shampoo

| | |
|---|---|
| Aqua | ad 100% |
| Lauryl Glucoside | 6,5% |
| Cocamidopropyl Betaine | 3,0% |
| Coco Glucoside | 2,5% |
| Rhamnolipid | 2,0% |
| Glyceryl Oleate | 1,5% |
| Sodium Cocoyl Glutamate | 1,0% |
| Panthenol | 0,5% |
| Polyquaternium-10 | 0,3% |
| Chamomilla Recutita Extract | 0,5% |
| Glyceryl Caprylate | 0,2% |
| Citric Acid | ad pH 5,5 |
| Parfum, Dyes | q.s. |

### Formulierung 50: Relaxing Good Sleep Bath

| | |
|---|---|
| Aqua | ad 100% |
| Cocamidopropyl Betaine | 3,0% |
| Coco Glucoside | 2,5% |
| Sodium Chloride | 2,5% |
| Disodium Lauryl Sulfosuccinate | 2,0% |
| Rhamnolipid | 2,0% |
| Glycerin | 1,7% |
| Sodium PCA | 0,5% |
| Glyceryl Oleate | 0,4% |
| Sodium Hydroxide | 0,3% |
| Citric Acid | ad pH 5,5 |
| Linalool | 0,1% |
| Limonene | 0,1% |
| Geraniol | 0,1% |
| Dyes | q.s. |

### Formulierung 51: Pampering Oil Bath

| | |
|---|---|
| Aqua | ad 100% |
| Glycine Soja Oil | 20,0% |
| Rhamnolipid | 5,0% |
| Polyglyceryl-3 Palmitate | 4,5% |
| Glyceryl Caprylate | 4,5% |
| Simmondsia Chinensis Oil | 1,5% |
| Prunus Amygdalus Dulcis (Sweet Almond) Oil | 1,0% |
| Triticum Vulgare Germ Oil | 1,0% |
| Tocopherol | 0,2% |
| Limonene | 0,1% |
| Linalool | 0,1% |
| Citral | 0,1% |
| Dyes | q.s. |

### Formulierung 51: Langzeit-Volumen-Shampoo

| | |
|---|---|
| Aqua | ad 100% |
| Sodium Laureth Sulfate | 7,0% |
| Cocamidopropyl Betaine | 3,0% |
| Coco Glucoside | 3,0% |
| Rhamnolipid | 2,0% |
| Nymphaea Coerulea Flower Extract | 2,0% |
| Hydrolyzed Rice Protein | 2,0% |
| Cocamide MEA | 2,0% |
| Polyquaternium-10 | 0,5% |
| Malic Acid | ad pH 5,5 |
| Disodium EDTA | 0,5% |
| Propylene Glycol | 0,5% |
| Benzophenone-4 | 0,3% |
| Ethylhexyl Methoxycinnamate | 0,3% |
| Sodium Chloride | 0,9% |
| Benzyl Alcohol | 0,9% |
| PPG-9 | 0,2% |
| Methylisothiazolinone | 0,1% |
| Methylparaben | 0,1% |
| Propylparaben | 0,1% |
| Parfum, Dyes | q.s. |

### Formulierung 52: Shampoo

| | |
|---|---|
| Aqua | ad 100% |
| Sodium Laureth Sulfate | 6,5% |
| Coco Glucoside | 5,0% |
| Cocamidopropyl Betaine | 3,0% |
| Rhamnolipid | 3,0% |
| Glycol Distearate | 2,8% |
| Dimethicone | 1,7% |
| Laureth-23 | 1,5% |
| Laureth-4 | 1,5% |
| Polyquaternium-7 | 0,5% |
| Cocamide MEA | 0,7% |
| Cetyl Alcohol | 0,4% |
| Simmondsia Chinensis Seed Oil | 0,8% |
| Panthenol | 0,3% |
| Malic Acid | ad pH 5,5 |
| Glycine | 0,7% |
| Benzyl Alcohol | 0,7% |
| Disodium EDTA | 0,5% |
| Sodium Hydroxide | 0,2% |
| Guar Hydroxypropyltrimonium Chloride | 0,5% |
| Ethylhexyl Methoxycinnamate | 0,7% |
| Methylparaben | 0,3% |
| Propylparaben | 0,3% |
| PPG-9 | 0,2% |
| Benzophenone-4 | 0,3% |
| Sodium Chloride | 0,8% |
| DMDM Hydantoin | 0,1% |
| Hexyl Cinnamal | 0,1% |
| Limonene | 0,1% |
| Dyes | q.s. |

### Formulierung 53: Shampoo

| | |
|---|---|
| Aqua | ad 100% |
| Sodium Laureth Sulfate | 5,0% |
| Sodium Lauryl Sulfate | 5,0% |
| Cocamidopropyl Betaine | 3,0% |
| Rhamnolipid | 2,5% |
| Olea Europaea Fruit Oil | 2,0% |
| Cocamide MEA | 2,0% |
| Sodium Chloride | 1,9% |
| Glycol Distearate | 1,7% |
| Benzyl Alcohol | 1,0% |
| Polyquaternium-10 | 0,5% |
| Hydroxypropyl Methylcellulose | 0,5% |
| Silicone Quaternium-18 | 0,9% |
| Malic Acid | ad pH 5,5 |
| Disodium EDTA | 0,5% |
| Sodium Xylenesulfonate | 0,7% |
| Trideceth-6 | 0,7% |
| Trideceth-12 | 0,5% |
| DMDM Hydantoin | 0,2% |
| Methylparaben | 0,3% |
| Propylparaben | 0,3% |
| Linalool | 0,1% |
| Hexyl Cinnamal | 0,1% |
| Butylphenyl Methylpropional | 0,1% |
| Limonene | 0,1% |
| Dyes | q.s. |

### Formulierung 54: Shampoo

| | |
|---|---|
| Aqua | ad 100% |
| Sodium Laureth Sulfate | 3,0% |
| Sodium Lauryl Sulfate | 3,5% |
| Glycol Distearate | 3,3% |
| Dimethicone | 3,0% |
| Cocamidopropyl Betaine | 2,5% |
| Rhamnolipid | 2,0% |
| Benzyl Alcohol | 0,7% |
| Betaine | 0,4% |
| Cetyl Alcohol | 0,7% |
| Cocamide MEA | 0,7% |
| Glyceryl Oleate | 0,5% |
| Propylene Glycol | 0,6% |
| Menthol | 0,7% |
| Guar Hydroxypropyltrimonium Chloride | 0,4% |
| Butylene Glycol | 0,5% |
| Menthyl Lactate | 0,2% |
| Disodium EDTA | 0,5% |
| Tocopheryl Acetate | 0,1% |
| Laureth-4 | 0,7% |
| Niacinamide | 0,1% |
| Maltodextrin | 0,2% |
| Sodium Ascorbyl Phosphate | 0,1% |
| PPG-9 | 0,2% |
| Salicylic Acid | 0,7% |
| Pyridoxine HCl | 0,1% |
| Malic Acid | ad pH 5,0 |
| Laureth-23 | 0,5% |
| Silica | 0,7% |
| Sodium Chloride | 0,7% |
| Sodium Hydroxide | 0,3 |
| Methylchloroisothiazolinone | 0,1% |
| Methylisothiazolinone | 0,1% |
| Parfum, Dyes | q.s. |

### Formulierung 55: Shower Cream

| | |
|---|---|
| Aqua | ad 100% |
| Glycerin | 7,5% |
| Glycine Soja Oil | 3,0% |
| Lauryl Glucoside | 3,0% |
| Sodium Coco Sulfate | 3,0% |
| Rhamnolipid | 2,5% |
| Alcohol | 1,5% |
| Xanthan Gum | 1,5% |
| Butyrospermum Parkii Butter | 1,2% |
| Sodium Cetearyl Sulfate | 1,0% |
| Sodium Cocoyl Glutamate | 1,0% |
| Disodium Cocoyl Glutamate | 1,0% |
| Tocopherol | 0,1% |
| Helianthus Annuus Seed Oil | 0,3% |
| Limonene | 0,1% |
| Benzyl Salicylate | 0,1% |
| Linalool | 0,1% |
| Dyes | q.s. |

### Formulierung 56: Shower Gel

| | |
|---|---|
| Aqua | ad 100% |
| Sodium Coco Sulfate | 5,0% |
| Glycerin | 5,0% |
| Lauryl Glucoside | 4,0% |
| Sodium Lactate | 2,5% |
| Rhamnolipid | 2,0% |
| Sodium Cocoyl Glutamate | 2,0% |
| Disodium Cocoyl Glutamate | 2,0% |
| Alcohol | 1,0% |
| Prunus Cerasus Fruit Extract | 1,0% |
| Limonene | 0,1% |
| Coumarin | 0,2% |
| Linalool | 0,1% |
| Citral | 0,1% |
| Dyes | q.s. |

### Formulierung 57: Liquid Soap

| | |
|---|---|
| Aqua | ad 100% |
| Glycerin | 7,0% |
| Alcohol | 4,0% |
| Sodium Coco Sulfate | 3,0% |
| Lauryl Glucoside | 2,5% |
| Rhamnolipid | 2,0% |
| Xanthan Gum | 1,5% |
| Mangifera Indica (Mango) Fruit Extract | 0,7% |
| Limonene | 0,1% |
| Linalool | 0,1% |
| Dyes | q.s. |

### Formulierung 58: Aroma Bath

| | |
|---|---|
| Aqua | ad 100% |
| Alcohol | 5,0% |
| Coco Glucoside | 5,0% |
| Sodium Coco Sulfate | 4,0% |
| Rhamnolipid | 2,5% |
| Xanthan Gum | 1,9% |
| Glycerin | 1,8% |
| Sodium Cocoyl Glutamate | 1,5% |
| Disodium Cocoyl Glutamate | 1,5% |
| Melissa Officinalis Extract | 1,0% |
| Lavandula Angustifolia Oil | 1,0% |
| Linalool | 0,1% |
| Limonene | 0,1% |
| Geraniol | 0,1% |
| Dyes | q.s. |

### Formulierung 59: Shampoo

| | |
|---|---|
| Aqua | ad 100% |
| Sodium Coco Sulfate | 6,0% |
| Lauryl Glucoside | 4,0% |
| Glycerin | 3,5% |
| Sodium Lactate | 2,5% |
| Rhamnolipid | 2,0% |
| Betaine | 0,8% |
| Sodium Chloride | 0,9% |
| Simmondsia Chinensis Seed Oil | 0,8% |
| Sodium Cocoyl Glutamate | 0,7% |
| Disodium Cocoyl Glutamate | 0,5% |
| Xanthan Gum | 0,5% |
| Hydrated Silica | 0,5% |
| Parfum, Dyes | q.s. |

### Formulierung 60: Shampoo for Children

| | |
|---|---|
| Aqua | ad 100% |
| Sodium Coco Sulfate | 7,0% |
| Decyl Glucoside | 5,0% |
| Lactis Proteinum | 3,5% |
| Sorbitan Caprylate | 3,0% |
| Rhamnolipid | 3,0% |
| Glycerin | 2,5% |
| Sodium Lactate | 2,5% |
| Alcohol | 2,0% |
| Hydrolyzed Wheat Protein | 0,7% |
| Hydrolyzed Wheat Starch | 0,7% |
| Sodium Chloride | 0,9% |
| Limonene | 0,1% |
| Citral | 0,1% |
| Phenethyl Alcohol | 0,1% |
| Dyes | q.s. |

### Formulierung 61: Shower Gel

| | |
|---|---|
| Aqua | ad 100% |
| Alcohol | 5,0% |
| Coco-Glucoside | 5,0% |
| Glycerin | 5,0% |
| Rhamnolipid | 2,5% |
| Xanthan Gum | 2,5% |
| Disodium Cocoyl Glutamate | 2,0% |
| Citric Acid | ad pH 5,0 |
| Citrus Aurantifolia (Lime) Oil | 1,0% |
| Sodium Cocoyl Glutamate | 1,0% |
| Glyceryl Oleate | 1,0% |
| Sodium Lauroyl Lactylate | 0,7% |
| Limonene | 0,1% |
| Citral | 0,1% |
| Linalool | 0,1% |
| Dyes | q.s. |

### Formulierung 62: Cream Soap

| | |
|---|---|
| Aqua | ad 100% |
| Alcohol | 5,0% |
| Coco Glucoside | 5,0% |
| Glycerin | 5,0% |
| Rhamnolipid | 2,5% |
| Disodium Cocoyl Glutamate | 2,0% |
| Xanthan Gum | 1,5% |
| Citric Acid | ad pH 5,5 |
| Malva Sylvestris Leaf Extract | 1,0% |
| Sodium Lauroyl Lactylate | 1,0% |
| Glyceryl Oleate | 1,0% |
| Sodium Cocoyl Glutamate | 0,7% |
| Linalool | 0,1% |
| Limonene | 0,1% |
| Dyes | q.s. |

### Formulierung 63: Caffeine Shampoo

| | |
|---|---|
| Aqua | ad 100% |
| Sodium Coco Sulfate | 6,0% |
| Glycerin | 5,5% |
| Lauryl Glucoside | 4,5% |
| Cocamidopropyl Betaine | 3,0% |
| Rhamnolipid | 2,5% |
| Lauroyl Sarcosine | 2,0% |
| Caffeine | 1,0% |
| Xanthan Gum | 1,0% |
| Hydroxypropyl Guar Hydroxypropyltrimonium Chloride | 0,3% |
| Alcohol | 0,8% |
| Coffea Arabica Bean Extract | 0,3% |
| Panthenyl Ethyl Ether | 0,4% |
| Panthenol | 0,2% |
| Limonene | 0,1% |
| Linalool | 0,1% |
| Dyes | q.s. |

### Formulierung 64: Shampoo

| | |
|---|---|
| Aqua | ad 100% |
| Sodium Laureth Sulfate | 6,5% |
| Cocamidopropyl Betaine | 3,0% |
| Sodium Chloride | 3,0% |
| Rhamnolipid | 2,0% |
| Panthenol | 0,2% |
| Benzophenone-4 | 0,9% |
| Glycol Distearate | 0,7% |
| Laureth-4 | 0,8% |
| Glycerin | 0,9% |
| Formic Acid | 0,2% |
| Decyl Glucoside | 0,9% |
| Polyquaternium-10 | 0,4% |
| Niacinamide | 0,1% |
| Sodium Benzoate | 0,1% |
| Potassium Sorbate | 0,2% |
| Citric Acid | ad pH 5,0 |
| Parfum, Dyes | q.s. |

### Formulierung 65: Pro-Vitamin Shampoo

| | |
|---|---|
| Aqua | ad 100% |
| Sodium Laureth Sulfate | 8,5% |
| Cocamidopropyl Betaine | 3,0% |
| Sodium Chloride | 2,0% |
| Disodium Cocoamphodiacetate | 2,0% |
| Rhamnolipid | 2,0% |
| Sodium Benzoate | 0,7% |
| Panthenol | 0,2% |
| Sodium Laureth-8 Sulfate | 0,7% |
| Citric Acid | ad pH 5,0 |
| Sodium Cocoyl Glutamate | 0,8% |
| Glycol Distearate | 0,9% |
| Styrene/Acrylates Copolymer | 0,5% |
| Sodium Oleth Sulfate | 0,7% |
| Coco Glucoside | 0,9% |
| Cocamide MEA | 0,7% |
| Isopropyl Alcohol | 0,2% |
| Parfum, Dyes | q.s. |

### Formulierung 66: Liquid Soap

| | |
|---|---|
| Aqua | ad 100% |
| Sodium Laureth Sulfate | 5,0% |
| Sodium Chloride | 3,5% |
| Cocamidopropyl Betaine | 3,5% |
| Coco Glucoside | 3,0% |
| Rhamnolipid | 2,0% |
| Glyceryl Oleate | 1,0% |
| Hibiscus Sabdariffa Flower Extract | 1,0% |
| Alcohol | 1,0% |
| Sodium Lactate | 1,0% |
| Citric Acid | ad pH 5,0 |
| Sodium Benzoate | 0,7% |
| Potassium Sorbate | 0,1% |
| Parfum, Dyes | q.s. |

### Formulierung 67: Oil Shower

| | |
|---|---|
| Aqua | ad 100% |
| Sodium Laureth Sulfate | 8,0% |
| Cocamidopropyl Betaine | 3,0% |
| Rhamnolipid | 2,0% |
| Acrylates Copolymer | 2,0% |
| Tocopheryl Acetate | 0,5% |
| PEG-7 Glyceryl Cocoate | 0,5% |
| Disodium EDTA | 0,4% |
| Benzophenone-4 | 0,3% |
| PEG-40 Hydrogenated Castor Oil | 0,7% |
| Cellulose | 0,7% |
| Hydroxypropyl Methylcellulose | 0,7% |
| Sodium Chloride | 0,9% |
| Ethylhexylglycerin | 0,2% |
| Phenoxyethanol | 0,7% |
| Methylisothiazolinone | 0,3% |
| Sodium Benzoate | 0,3% |
| Sodium Hydroxide | 0,3% |
| Citric Acid | ad pH 5,5 |
| Parfum, Dyes | q.s. |

### Formulierung 68: Shampoo

| | |
|---|---|
| Aqua | ad 100% |
| Sodium Laureth Sulfate | 9,0% |
| Cocamidopropyl Betaine | 3,0% |
| Sodium Chloride | 2,5% |
| Glycol Distearate | 2,5% |
| Hydrolyzed Keratin | 2,0% |
| Simmondsia Chinensis (Jojoba) Seed Oil | 2,0% |
| Rhamnolipid | 2,0% |
| Argania Spinosa Kernel Oil | 0,5% |
| Persea Gratissima (Avocado) Oil | 0,5% |
| Rosmarinus Officinalis (Rosemary) Leaf Extract | 0,3% |
| Helianthus Annuus (Sunflower) Seed Oil | 0,3% |
| Cocamide MEA | 0,4% |
| Guar Hydroxypropyltrimonium Chloride | 0,2% |
| Polyquaternium-47 | 0,2% |
| PEG-20 Castor Oil | 0,5% |
| Phenethyl Benzoate | 0,7% |
| Disodium EDTA | 0,2% |
| Methylchloroisothiazolinone | 0,3% |
| Methylisothiazolinone | 0,3% |
| Benzyl Salicylate | 0,1% |
| Hexyl Cinnamal | 0,1% |
| Citronellol | 0,1% |
| Dyes | q.s. |

### Formulierung 69: Foam Shampoo

| | |
|---|---|
| Aqua | ad 100% |
| Sodium Laureth Sulfate | 5,0% |
| Isobutane | 5,0% |
| Decyl Glucoside | 5,0% |
| Propane | 3,5% |
| Sodium Cocoamphoacetate | 3,5% |
| Rhamnolipid | 3,5% |
| Panthenol | 0,2% |
| Polyquaternium-10 | 0,2% |
| Hydroxypropyl Guar Hydroxypropyltrimonium Chloride | 0,1% |
| Sodium PCA | 0,5% |
| Octyldodecyl PCA | 0,5% |
| Maltodextrin | 0,5% |
| Tilia Cordata Flower Extract | 0,7% |
| Sodium Chloride | 0,9% |
| Citric Acid | ad pH 5,0 |
| Glycerin | 0,9% |
| Propylene Glycol | 0,3% |
| Butane | 0,7% |
| Sodium Acetate | 0,7% |
| Sorbitol | 0,5% |
| Isopropyl Alcohol | 0,5% |
| Dyes | q.s. |

### Formulierung 70: Anti-Dandruff Shampoo

| | |
|---|---|
| Aqua | ad 100% |
| Sodium Laureth Sulfate | 10,0% |
| Cocamidopropyl Betaine | 3,0% |
| Acrylates Copolymer | 2,5% |
| Sodium Lauroyl Glutamate | 2,5% |
| Zinc Pyrithione | 2,0% |
| Rhamnolipid | 2,0% |
| Piroctone Olamine | 0,7% |
| Hydroxypropyl Guar Hydroxypropyltrimonium Chloride | 0,2% |
| Propylene Glycol | 0,5% |
| Sodium PCA | 0,2% |
| Citrus Medica Limonum (Lemon) Peel Extract | 0,5% |
| Citric Acid | ad pH 5,0 |
| Glycol Distearate | 0,8% |
| PPG-9 | 0,2% |
| Laureth-4 | 0,2% |
| Sodium Chloride | 0,9% |
| Sodium Hydroxide | 0,3% |
| Sodium Naphthalenesulfonate | 0,7% |
| Sorbitol | 0,7% |
| DMDM Hydantoin | 0,1% |
| Sodium Benzoate | 0,8% |
| Limonene | 0,1% |
| Linalool | 0,1% |
| Butylphenyl Methylpropional | 0,1% |
| alpha-Isomethyl lonone | 0,1% |
| Parfum, Dyes | q.s. |

### Formulierung 71: After-Sun Hair & Body Shampoo

| | |
|---|---|
| Aqua | ad 100% |
| Sodium Laureth Sulfate | 7,0% |
| Cocamidopropyl Betaine | 5,0% |
| PEG-18 | 3,5% |
| Glyceryl Oleate | 3,0% |
| Rhamnolipid | 3,0% |
| Glyceryl Cocoate | 2,5% |
| Glycol Distearate | 2,5% |
| Coco Glucoside | 2,0% |
| Passiflora Incarnata Seed Oil | 1,5% |
| Laureth-4 | 1,5% |
| Panthenol | 0,3% |
| Sodium Chloride | 0,9% |
| Sodium PCA | 0,7% |
| Polyquaternium-10 | 0,5% |
| Octyldodecyl PCA | 0,2% |
| Glycerin | 0,9% |
| PPG-9 | 0,7% |
| Citric Acid | ad pH 5,5 |
| Tetrasodium EDTA | 0,3% |
| Quaternium-80 | 0,2% |
| Propylene Glycol | 0,3% |
| Isopropyl Alcohol | 0,3% |
| Sodium Acetate | 0,5% |
| Sodium Benzoate | 0,7% |
| Limonene | 0,1% |
| Linalool | 0,1% |
| Benzyl Salicylate | 0,1% |
| Butylphenyl Methylpropional | 0,1% |
| Dyes | q.s. |

### Formulierung 72: Shower Gel

| | |
|---|---|
| Aqua | ad 100% |
| Sodium Laureth Sulfate | 8,5% |
| Cocamidopropyl Betaine | 3,0% |
| Sodium Chloride | 2,7% |
| Rhamnolipid | 2,5% |
| Prunus Cerasus (Bitter Cherry) | 2,0% |
| Polyquaternium-7 | 0,5% |
| Bisabolol | 0,2% |
| Trideceth-9 | 0,5% |
| Sodium Sulfate | 0,9% |
| Tetrasodium EDTA | 0,4% |
| Citric Acid | ad pH 5,5 |
| Sodium Benzoate | 0,7% |
| Benzyl Alcohol | 0,7% |
| Benzyl Salicylate | 0,1% |
| Geraniol | 0,2% |
| Hexyl Cinnamal | 0,1% |
| Limonene | 0,1% |
| Linalool | 0,1% |
| Dyes | q.s. |

### Formulierung 73: Liquid Soap

| | |
|---|---|
| Aqua | ad 100% |
| Sodium Laureth Sulfate | 8,0% |
| Cocamidopropyl Betaine | 3,0% |
| Rhamnolipid | 2,5% |
| Sodium Benzoate | 1,0% |
| Lactic Acid | 0,7% |
| Simmondsia Chinensis Oil | 0,5% |
| Chamomilla Recutita Extract | 0,5% |
| Sodium Chloride | 0,9% |
| Sodium Lactate | 0,7% |
| Styrene/Acrylates Copolymer | 0,5% |
| Glycerin | 0,5% |
| Polyquaternium-7 | 0,3% |
| Hexyl Cinnamal | 0,1% |
| Sorbitol | 0,2% |
| Trisodium Ethylenediamine Disuccinate | 0,2% |
| Butylphenyl Methylpropional | 0,2% |
| Propylene Glycol | 0,3% |
| PEG-40 Hydrogenated Castor Oil | 0,9% |
| Trideceth-9 | 0,7% |
| 1,2-Hexanediol | 0,7% |
| Caprylyl Glycol | 0,4% |
| Dyes | q.s. |

### Formulierung 74: Cream Soap

| | |
|---|---|
| Aqua | ad 100% |
| Sodium Laureth Sulfate | 8,0% |
| Cocamidopropyl Betaine | 3,0% |
| Sodium Chloride | 2,6% |
| Rhamnolipid | 2,5% |
| Polyquaternium-7 | 0,4% |
| Glycerin | 0,9% |
| Glycol Distearate | 0,7% |
| Laureth-4 | 0,5% |
| Laureth-2 | 0,8% |
| PEG-55 Propylene Glycol Oleate | 0,4% |
| Propylene Glycol | 0,5% |
| Citric Acid | ad pH 5,5 |
| Hexyl Cinnamal | 0,1% |
| Benzyl Salicylate | 0,1% |
| Linalool | 0,1% |
| Limonene | 0,1% |
| Butylphenyl Methylpropional | 0,1% |
| Sodium Benzoate | 0,1% |
| Dyes | q.s. |

**Tabelle 1: Weitere Formulierungsbeispiele**

| | **76** | **77** | **78** | **79** | **80** | **81** | **82** | **83** | **84** | **85** |
|---|---|---|---|---|---|---|---|---|---|---|
| **Wasser** | ad 100% | | | | | | | | | |
| **Produktbeispiel 1: Rhamnolipid** | 9,0% | 15,0% | 5,5% | 5,0% | 7,0% | 3,0% | 6,0% | 4,0% | 3,5% | 3,0% |
| **Sodium Laureth Sulfate** | - | - | - | 7,0% | - | - | - | 4,5% | - | - |
| **Sodium Lauryl Sulfate** | - | - | - | - | -5,0% | - | - | - | -3,5% | 1,0% |
| **Cocamidopropyl Betaine** | - | - | - | - | - | 6,0% | - | 3,0% | 2,0% | 4,5% |
| **Sodium Cocoamphoacetate** | - | - | - | - | - | - | - | -3,5% | | - |
| **Lauryl Glucoside** | - | - | - | - | - | | 2,0% | - | - | - |
| **Coco Glucoside** | - | - | - | - | - | - | 5,5% | 1,5% | - | - |
| **Sodium Cocoyl Glutamate** | - | - | - | - | - | - | 0,7% | - | - | - |
| **Stearic Acid** | - | - | - | - | - | - | - | 1,0% | - | 3,5% |
| **Glyceryl Glucoside** | - | 0,5% | - | - | 0,3% | - | 0,2% | - | - | - |
| **Sucrose Cocoate** | 0,5% | -1,0% | 1,0% | 1,0% | 1,0% | 0,5% | - | 1,0% | 1,0% | 1,0% |
| **Glycerin** | - | 1,0% | 0,5% | - | - | 0,3% | 1,5% | 1,0% | - | 1,0% |
| **PEG-7 Glyceryl Cocoate** | - | 0,7% | - | 0,5% | - | - | - | - | - | 0,5% |
| **Trideceth-9** | - | - | - | 0,2% | - | - | - | 0,3% | - | - |
| **Polyglyceryl-4 Caprate** | - | - | 1,0% | - | 0,4% | - | 0,5% | - | - | - |
| **Polyquaternium-10** | - | 0,2% | - | 0,1% | - | - | - | 0,2% | - | - |
| **Hydroxypropyl Guar Hydroxypropyltrimonium Chloride** | 0,2% | - | 0,3% | 0,2% | 0,2% | 0,2% | 0,3% | 0,1% | 0,2% | - |
| **Silicone Quaternium-22** | - | - | - | 0,5% | - | - | - | 0,5% | - | - |
| **Dimethicone** | - | 0,5% | - | - | - | - | - | 1,0% | - | - |
| **Amodimethicone** | - | 0,1% | - | - | 1,0 % | 0,3% | - | - | 0,5% | - |
| **Argania Spinosa Oil** | - | - | 0,2% | 0,1% | - | 0,1% | 0,2% | - | - | - |
| **Glycol Distearate** | 0,5% | 0,5% | - | - | 0,5% | - | 0,3% | 0,5% | | 0,5% |
| **Acrylates / C10-30 Alkyl Acrylate Crosspolymer** | 1,0% | - | - | - | 0,5% | - | - | - | 0,5% | - |
| **Sodium Hydroxide, 25%** | 1,5% | - | - | - | 0,8% | - | - | - | 0,7% | - |
| **Isostearamide MIPA; Glyceryl Laurate** | - | - | - | - | - | 2,5% | - | - | 1,0% | 0,5% |
| **Cocamide DEA** | - | - | - | 1,0% | 0,5% | - | - | - | - | - |
| **Sodium Chloride** | 0,1% | 1,2% | - | 0,5% | - | 1,5% | - | 2,0% | - | 1,2% |
| **PEG-120 Methyl Glucose Dioleate** | - | 3,5% | - | 0,2% | - | - | - | 0,6% | - | - |
| **Xanthan Gum** | - | - | 1,2% | - | - | 0,2% | 1,5% | - | - | - |
| **Cellulose** | - | - | - | 0,1% | - | 0,1% | 0,1% | - | 0,1% | - |
| **Zinc Pyrithione** | - | 0.1% | - | 1.1% | - | - | - | 0.5% | - | - |
| **Benzophenone-4** | - | 0,1% | - | 0,2% | - | - | - | 0,2% | 0,1% | - |
| **Tetrasodium EDTA** | 0,1% | 0,1% | - | 0,1% | - | - | - | 0,1% | 0,1% | - |
| **Caffeine** | - | - | 0,1% | - | - | - | - | 0,1% | 0,1% | - |
| **Hydrolyzed Keratin** | - | - | 0,1% | 0,1% | - | 0,1% | 0,2% | - | 0,1% | - |
| **Panthenol** | 0,1% | 0,1% | 0,1% | 0,1% | 0,1% | 0,1% | | 0,1% | 0,1% | 0,1% |
| **Citric Acid** | ad pH 5,5 | | | | | | | | | |
| **Parfums, Dyes, Preservatives** | q.s. | | | | | | | | | |

**Tabelle 2: Weitere Formulierungsbeispiele**

| | **86** | **87** | **88** | **89** | **90** | **91** | **92** | **93** | **94** | **95** |
|---|---|---|---|---|---|---|---|---|---|---|
| **Wasser** | ad 100% | | | | | | | | | |
| **Produktbeispiel 1: Rhamnolipid** | 12,0 % | 7,0% | 5,0% | 4,0% | 7,0% | 2,5% | 6,0% | 4,0% | 3,0% | 3,0% |
| **Sodium Laureth Sulfate** | - | - | 8,0% | - | - | - | - | 3,5% | - | - |
| **Coco-Betaine** | - | - | - | 5,5% | 5,0% | - | - | 3,0% | - | - |
| **Cocamidopropyl Betaine** | - | - | - | - | - | 6,0% | - | - | 3,0% | 4,0% |
| **Sodium Cocoamphoacetate** | - | - | - | - | 2,0% | - | - | - | - | - |
| **Disodium Lauryl Sulfosuccinate** | - | - | - | - | - | 1,5% | - | - | - | 2,0% |
| **Coco Glucoside** | - | - | - | - | 2,0% | - | 4,0% | 1,5% | 6,0% | - |
| **Sodium Cocoyl Glutamate** | - | - | - | - | - | - | 2,5% | 0,5% | 2,5% | 0,3% |
| **Stearic Acid** | - | 8,0% | - | - | - | - | - | - | - | 4,5% |
| **Sodium Cocoyl Glycinate** | - | - | - | - | - | 3,0% | 0,5% | - | - | 0,3% |
| **Sodium Lauroyl Methyl Isethionate** | - | - | - | - | - | 1,0% | - | 1,0% | 2,0% | 0,5% |
| **Sucrose Cocoate** | 0,5% | 1,1% | - | 1,0% | 1,0% | - | - | 0,3% | 1,0% | 0,3% |
| **Glycerin** | 1,5% | 0,3% | 0,5% | - | 0,3% | 0,5% | 1,0% | 0,5% | - | 1,0% |
| **PEG-7 Glyceryl Cocoate** | - | - | 0,5% | - | - | 0,8% | - | 0,5% | - | 0,5% |
| **PEG-40 Hydrogenated Castor Oil** | - | - | 0,5% | - | - | 1,5% | - | 0,3% | - | - |
| **Polyglyceryl-4 Caprate** | 0,5% | - | - | - | 0,4% | - | 0,5% | - | 1,1% - | - |
| **Polyquaternium-11** | - | 0,2% | - | - | 0,1% | 0,3% | - | 0,2% | - | - |
| **Guar Hydroxypropyltrimonium Chloride** | 0,3% | - | 0,3% | 0,2% | 0,2% | - | 0,3% | 0,1% | 0,4% | 0,2% |
| **Silicone Quaternium-22** | - | - | 0,5% | - | - | - | - | 1,5% | - | - |
| **PEG-12 Dimethicone** | - | - | - | - | - | 1,5% | - | - | - | - |
| **Dimethicone** | - | - | 1,0% | - | 0,5% | - | - | - | - | - |
| **Aminopropyl Dimethicone** | - | - | - | - | - | 0,3% | - | - | - | - |
| **Helianthus Annuus Oil** | 0,1% | - | 0,2% | 0,1% | - | 0,1% | 0,3% | 0,2% | - | 0,1% |
| **PEG-3 Distearate** | - | - | 0,5% | - | - | - | 0,8% | - | 0,5% | - |
| **Acrylates** / **C10-30 Alkyl Acrylate Crosspolymer** | - | - | 0,2% | - | 0,5% | - | - | - | - | - |
| **Sodium Hydroxide, 25%** | - | - | 0,3% | - | 0,8% | - | - | - | - | - |
| **Cocamide DEA** | - | - | - | - | - | 2,5% | - | - | - | 0,5% |
| **Cocamide MEA** | - | - | - | 1,0% | - | - | - | 1,0% | - | - |
| **Sodium Chloride** | 0,4% | 0,7% | 0,1% | - | - | 1,5% | - | 2,0% | - | 1,2% |
| **Propylene Glycol (and) PEG-55 Propylene Glycol Oleate** | - | 2,5% | 2,0% | - | - | 0,3% | - | 0,6% | - | 0,2% |
| **Xanthan Gum** | 1,2% | - | - | 1,0% | 0,2% | - | 1,5% | 0,1% | 1,1% | - |
| **Hydroxyethyl Cellulose** | 0,1% | - | - | 0,1% | - | 0,1% | 0,1% | - | - | - |
| **Benzophenone-4** | - | 0,1% | 0,2% | - | - | 0,2% | - | 0,2% | - | 0,1% |
| **Menthol** | 0,1% | - | - | - | - | - | - | - | - | 0,1% |
| **Tetrasodium Glutamate Diacetate** | - | 0,1% | - | 0,1% | 0,1% | - | 0,1% | 0,1% | - | 0,1% |
| **Caffeine** | 0,1% | - | 0,1% | - | - | - | - | 0,1% | - | - |
| **Coumarin** | 0,1% | - | - | 0,1% | 0,1% | - | 0,1% | 0,1% | - | 0,1% |
| **Hydrolyzed Wheat Protein** | 0,1% | - | 0,1% | 0,1% | - | - | 0,2% | 0,1% | 0,1% | 0,1% |
| **Panthenol** | 0,1% | 0,1% | - | 0,1% | 0,1% | 0,1% | 0,1% | - | 0,1% | 0,1% |
| **Citric Acid** | ad pH 5,5 | | | | | | | | | |
| **Parfums, Dyes, Preservatives** | q.s. | | | | | | | | | |

**Tabelle 3: Weitere Formulierungsbeispiele**

| | **96** | **97** | **98** | **99** | **100** | **101** | **102** | **103** | **104** | **105** |
|---|---|---|---|---|---|---|---|---|---|---|
| **Wasser** | ad 100% | | | | | | | | | |
| **Produktbeispiel 1: Rhamnolipid** | 9,5% | 3,0% | 4,0% | 4,0% | 3,0% | 4,0% | 6,0% | 4,0% | 5,0% | 4,5% |
| **MIPA-Laureth Sulfate** | - | 5,0% | - | - | - | - | - | - | - | - |
| **Sodium C14-16 Olefin Sulfonate** | - | - | 7,0% | - | - | 3,0% | 1,5% | - | - | - |
| **Coco-Betaine** | - | - | - | 5,5% | - | - | - | - | 5,0% | - |
| **Cocamidopropyl Betaine** | - | - | 2,0% | - | 5,0% | 5,0% | 2,0% | 2,5% | - | - |
| **Sodium Cocoamphopropionate** | - | - | 1,0% | - | 2,0% | - | - | - | - | - |
| **Disodium Laureth Sulfosuccinate** | - | - | 1,0% | - | 2,5% | 1,0% | - | 1,5% | - | 2,0% |
| **Coco Glucoside** | - | - | - | 2,0% | - | - | 3,0% | - | 5,0% | - |
| **Sodium Cocoyl Glutamate** | - | - | - | 1,5% | - | 1,5% | 1,5% | - | - | 0,3% |
| **Lauric Acid** | - | - | - | - | 3,0% | - | - | 8,0% | - | 4,5% |
| **Sodium Cocoyl Glycinate** | - | - | -1,5% | 1,5% | - | - | 0,5% | 0,5% | - | - |
| **Sodium Cocoyl Sarcosinate** | - | - | 1,0% | - | - | - | 0,5% | - | - | - |
| **Dicaprylylether** | 0,5% | 1,1% | - | - | - | - | 0,3% | - | - | - |
| **Glycerin** | 0,5% | 0,3% | 0,5% | 1,5% | - | 0,5% | 1,0% | 0,5% | 0,3% | 1,0% |
| **PEG-7 Glyceryl Cocoate** | - | 1,2% | - | - | 0,5% | 0,8% | - | - | - | - |
| **Polysorbate 20** | - | 0,3% | - | - | 0,5% | - | - | - | - | - |
| **Polyglyceryl-4 Laurate** | 0,5% | - | - | 0,3% | - | - | 0,5% | - | 1,1% | - |
| **Polyquaternium-37** | - | - | 0,2% | - | - | - | - | - | - | - |
| **Hydroxypropyl Guar Hydroxypropyltrimonium Chloride** | - | - | 0,1% | 0,2% | - | - | 0,3% | - | 0,3% | - |
| **Cassia Hydroxypropyltrimonium Chloride** | 0,3% | - | - | - | - | 0,2% | - | - | - | - |
| **Sodium Hydroxypropyl Starch Phosphate** | - | - | - | - | 0,3% | - | - | 0,5% | - | - |
| **Hydroxypropyl** | 0,2% | - | - | 0,4% | 0,2% | - | - | 0,2% | 0,2% | |
| **Methylcellulose** | | | | | | | | | | |
| **Dimethicone** | - | - | - | - | - | 1,0% | - | - | - | - |
| **Aminopropyl Dimethicone** | - | - | - | - | - | 0,5% | - | - | - | 0,5% |
| **Palmitamidopropyltrimoniu m Chloride** | - | - | - | - | - | 0,5% | 0,2% | - | - | - |
| **Persea Gratissima (Avocado) Oil** | - | 10,0% | 0,2% | 0,1% | 0,2% | - | 0,3% | - | 0,1% | 0,1% |
| **Glycol Distearate** | - | - | 0,5% | - | 0,5% | 0,8% | - | - | - | 0,3% |
| **Carbomer** | - | - | - | - | - | 0,5% | 0,7% | - | - | - |
| **Sodium Hydroxide, 25%** | - | - | - | - | - | 0,7% | 1,0% | - | - | - |
| **Isostearamide MIPA; Glyceryl Laurate** | - | - | 0,8% | - | 0,3% | - | - | 0,4% | - | - |
| **Sorbitan Sesquicaprylate** | - | - | 0,2% | 1,0% | - | - | 0,4% | 1,0% | 0,5% | 0,7% |
| **Sodium Chloride** | 0,4% | -0,3% | 0,3% | 0,2% | 0,5% | - | - | 2,0% | 0,5% | - |
| **PEG-18 Glyceryl Oleate/Cocoate** | - | - | - | - | 1,2% | 0,3% | - | - | - | - |
| **Xanthan Gum** | - | - | - | 1,0% | - | - | - | - | - | 0,2% |
| **Alginat** | 0,2% | - | - | 0,1% | - | - | - | - | 1,2% | - |
| **Caragenaan** | 0,8% | - | - | - | - | - | - | - | - | 1,0% |
| **Silica** | - | - | 0,1% | - | - | 0,2% | - | - | - | - |
| **Cetearyl Alcohol** | - | 0,2% | - | - | 0,3% | - | - | - | - | - |
| **Benzophenone-4** | - | - | 0,2% | - | 0,2% | 0,2% | - | - | - | 0,1% |
| **Tetrasodium EDTA** | 0,1% | 0,1% | - | 0,1% | 0,1% | - | 0,1% | 0,1% | - | 0,1% |
| **Octopirox** | - | - | 0,1% | - | - | 0,1% | - | - | - | - |
| **Zinc PCA** | - | - | - | - | 0,1% | - | - | - | - | 0,1% |
| **Creatine** | 0,1% | - | - | 0,1% | - | - | 0,1% | 0,1% | - | 0,1% |
| **Hydrolyzed Collagen** | 0,1% | - | 0,1% | 0,1% | - | - | 0,2% | 0,1% | 0,1% | 0,1% |
| **Salicylic Acid** | - | - | 0,1% | - | 0,1% | 0,1% | - | - | - | 0,1% |
| **Panthenol** | 0,1% | 0,1% | - | 0,1% | 0,1% | 0,1% | 0,1% | - | 0,1% | 0,1% |
| **Lactic Acid** | - | - | 0,2% | - | - | 0,5% | 0,3% | - | - | - |
| **PEG-14M** | - | 0,1% | - | - | 0,2% | - | - | - | - | - |
| **1,2-Hexanediol** | 0,3% | 0,2% | - | - | - | 0,3% | - | 0,2% | - | - |
| **Citric Acid** | ad pH 5,5 | | | | | | | | | |
| **Parfums, Dyes, Preservatives** | q.s. | | | | | | | | | |

**Tabelle 4: Liste der eingesetzten Rohstoffe**

| **INCl** | **Handelsname, Firma** |
|---|---|
| 1,2-Hexanediol | Hydrolite-6 841129, Symrise |
| 2-Oleamido-1,3-Octadecanediol | Mexanyl GZ, Chimex |
| Acrylates / C10-30 Alkyl Acrylate Crosspolymer | TEGO Carbomer 341 ER, Evonik Industries AG, 100% |
| Acrylates Copolymer | Eudragit FS 30 D, The Dow Chemical Company |
| Acrylates/C10-30 Alkyl Acrylate Crosspolymer | TEGO Carbomer 350 DER, 341 ER & 750 HD, Evonik Industries AG |
| Alcohol | Koei 4, Koei Science |
| Algin | Hydagen 558 P, BASF Personal Care and Nutrition Gmbh |
| Aloe Barbadensis Leaf Juice | Aloe-Con UP 40, Florida Food Products Inc. |
| alpha-Isomethyl lonone | alpha-Isomethylionone, Chemos GmbH |
| Amodimethicone | DC 949, Dow Corning, 100% |
| Amyl Cinnamal | Seridefrizz Intense, Cheemyunion Quimica Ltda. |
| Argania Spinosa Oil (Argania Spinosa Kernel Oil) | Argan Oil, DSM Nutritional Products Ltd. |
| Arginine | L-Arginine, Arjinomoto U.S.A. Inc. |
| Benzophenone-4 | Uvinul MS 40, BASF Corporation |
| Benzyl Alcohol | Microcare® BNA, THOR PERSONAL CARE SAS |
| Benzyl Benzoate | Morflex Benzyl Benzoate, Morflex Inc. |
| Benzyl Salicylate | Seridefrizz Intense, Cheemyunion Quimica Ltda. |
| Betaine | TEGO Natural Betaine, Evonik Industries AG, 100% |
| BHT | lonol, Shell Chemical Company |
| Bisabolol | Hydrogen B, BASF Personal Care & Nutrition GmbH |
| Butane | |
| Butyl Methoxydibenzoylmethane | Eusolex 9020, Merck KGaA |
| Butylene Glycol | Butylene Glycol, Oxea Corparation |
| Butylparaben | s&m butylparaben, Schülke & Mayr GmbH |
| Butylphenyl Methylpropional | Lily Aldehyde, LC United Chemical Corp. |
| Butyrospermum Parkii Butter Extract | Cosmosil 600, International Cosmetic Science Centre |
| C11-15 Pareth-7 | Tergitol 15-S-7 Surfactant, The Dow Chemical Company |
| C12-13 Pareth-23 | Brij LT23, Croda Europe, Ltd. |
| C12-13 Pareth-3 | Brij LT3, Croda Europe, Ltd. |
| C12-15 Pareth-12 | Mulsifan RT 203/80, Zschimmer & Schwarz GmbH & Co KG |
| Caffeine | Caffeine, Merck KGaA/EMD Chemicals, Inc. |
| Camellia Oleifera Seed Oil | Camellia Sasanqua Oil, Ikeda Corporation |
| Camellia Sinensis Leaf Extract | Green Tea Extract CG, Sabinsa Corporation |
| Caprylyl Glycol | sensiva® SC 10 |
| Caprylyl/Capryl Glucoside | Plantacare 810 UP, BASF Cognis |
| Carbomer | TEGO Carbomer 140, Evonik Industries AG, 100% |
| Cassia Hydroxypropyltrimonium Chloride | Formularbeginn Sensomer ST 250-Polymer, Lubrizol Formularende |
| Cellulose | Arbocel A300, J. Rettenmaier & Söhne |
| Ceteareth-25 | TEGINACID C, Evonik Industries AG, 100% |
| Cetearyl Alcohol | TEGO Alkanol 1618, Evonik Industries AG, 100% |
| Cetrimonium Chlorid | VARISOFT 300, Evonik Industries AG, 30% |
| Chamomilla Recutita (Matricaria) Extract | Recentia CR, AkzoNobel Global Personal Care |
| Chamomilla Recutita (Matricaria) Flower Extract | Recentia Chamomilla Recutita Flower Zeta Fraction, AkzoNobel Global Personal Care |
| Chondrus Crispus (Carrageenan) | Gelcarin PC 379, Azelis |
| Cinnamyl Alcohol | Hexatrate, Vevy Europe SpA |
| Citral | Citral FF, Symrise AG |
| Citric Acid | Citric Acid USP Granular, DSM Nutritional Products, Inc. |
| Citronellol | Seridefrizz Intense Salon, Chemyunion Química Ltda. |
| Citrus Aurantifolia (Lime) Oil | AEC Lime Oil, A & E Connock, Perfumery & Cosmetics Ltd. |
| Citrus Medica Limonum (Lemon) Peel Extract | OriStract LPE, Orient Stars LLC Formularende |
| Cocamide DEA | REWOMID DC 212 S, Evonik Industries AG, 100% |
| Cocamide MEA | REWOMID D 212, Evonik Industries AG, 100% |
| Cocamide MIPA | Rewomid IPP 240, Evonik Goldschmidt GmbH |
| Cocamidopropyl Betaine | TEGO Betain CK D, Evonik Industries AG, 82% |
| Cocamidopropyl Betaine | TEGO Betain F 50, Evonik Industries AG, 38% |
| Cocamidopropyl Hydroxysultaine | Mirataine CBS, Rhodia Inc. |
| Coco Glucoside | Plantacare 818 UP, BASF Cognis, 51% |
| Coco-Betaine | Dehyton AB 30, BASF Cognis, 31% |
| Cocodimonium Hydroxypropyl Hydrolyzed Keratin | Croquat WKP, Croda Europe, Ltd. |
| Cocos Nucifera Oil/Coconut Oil | AEC Coconut Oil RD, A & E Connock, Perfumery & Cosmetics Ltd. |
| Coffea Arabica Bean Extract | Chlorogenic Acid 50%, Sabinsa Corporation |
| Coumarin | Rhodiascent extra pure, Rhodia Organics |
| Creatine | TEGO Cosmo C 100, Evonik Industries AG, 100% |
| Decyl Glucoside | Plantacare 2000 UP, BASF Cognis |
| Denatonium Benzoate | AEC Denatonium Benzoate, A & E Connock, Perfumery & Cosmetics Ltd. |
| Diamond Powder | AC Diamond Dust, Active Concepts LLC |
| Dicaprylylether | Cetiol OE, BASF Cognis |
| Dimethicone | DC 200 Fluid 100 cSt, Dow Corning, 100% |
| Dimethicone (And) Dimethiconol | DC 1503 Fluid, Dow Corning |
| Dimethiconol | Dow Corning 1-1254 Fluid, Dow Corning Corporation |
| Disodium Cocoamphodiacetate | Rewoteric AM 2 C NM, Evonik Goldschmidt GmbH |
| Disodium Cocoyl Glutamate | Planatpon ACG LC, BASF Cognis |
| Disodium Edta | Dissolvine NA-2-P, AkzoNobel Global Personal Care |
| Disodium Laureth Sulfosuccinate | REWOPOL SB FA 30 B, Evonik Industries AG, 40% |
| Disodium Lauroamphodiacetate | Mackam 2L, Rhodia Inc. |
| Disodium Lauryl Sulfosuccinate | REWOPOL SB F 12 P, Evonik Industries AG, 95% |
| Divinyldimethicone/Dimethicone Copolymer | Dow Corning 2-1998 Anionic Emulsion, Dow Corning Corporation |
| Ethylhexyl Methoxycinnamate | Parsol MCX, DSM Nutritional Products, Inc. |
| Ethylhexyl Salicylate | Parsol EHS, DSM Nutritional Products, Inc. |
| Ethylhexylglycerin | Sensiva SC 50, Schulke & Mayr GmbH |
| Ethylparaben | s&m ethylparaben, Schülke & Mayr GmbH |
| Formic Acid | euxyl® K 600, Schülke & Mayr GmbH |
| Geraniol | Nerol 800, International Flavors & Fragrances Inc. |
| Gluconolactone | Lysactone, Roquette America, Inc. |
| Glycerin | Glycerol EP, vegetable, Spiga Nord, 99,7% |
| Glyceryl Caprylate | Dermosoft GMCY, Dr. Straetmans |
| Glyceryl Cocoate | Imwitor 928, Sasol Germany GmbH |
| Glyceryl Glucoside | Hydagen GG, BASF Cognis |
| Glyceryl Linoleate | AEC Glyceryl Linoleate, A & E Connock, Perfumery & Cosmetics Ltd. |
| Glyceryl Linolenate | Vitamin F Glyceryl Ester CLR, CLR |
| Glyceryl Oleate | Tegin O V, Evonik Goldschmidt GmbH |
| Glycine | AEC Glycine, A & E Connock, Perfumery & Cosmetics Ltd. |
| Glycine Soja (Soybean) Oil | Cropure Soybean, Croda Europe, Ltd. |
| Glycol Distearate | TEGIN G 1100, Evonik Industries AG, 100% |
| Guar Hydroxypropyltrimonium Chloride | Cosmedia Guar C 261, BASF Personal Care and Nutrition Gmbh / Jaguar C-17, Rhodia Inc. und andere |
| Helianthus Annuus (Sunflower) Oil | ABS Sunflower Extract BG, Active Concepts LLC |
| Helianthus Annuus (Sunflower) Seed Oil | Jeen Sunflower Oil, Jeen International Corporation |
| Helianthus Annuus Seed Oil | AEC Sunflower Oil, A & E Connock, Perfumery & Cosmetics Ltd. |
| Hexyl Cinnamal | Seridefrizz Intense Home Care, Chemyunion Química Ltda. |
| Hexyl Salicylate | Hexyl Salicylate, International Flavors & Fragrances, Inc. |
| Hexylene Glycol | Hexasol, Arkema |
| Hibiscus Sabdariffa Flower Extract | Hibiscus Infusion, Lush LTD |
| Honey (Mel) | Honey 150-L, Naturex AG |
| Hydrated Silica | Sipernat 22, Evonik Degussa GmbH |
| Hydrochloric Acid | |
| Hydrogenated Castor Oil | Cutina HR Powder, BASF Personal Care and Nutrition Gmbh |
| Hydrogenated Coconut Acid | Prifac 5900, Croda Europe, Ltd. |
| Hydrogenated Palm Glycerides Citrate | Controx KS C, BASF Personal Care and Nutrition Gmbh |
| Hydrolyzed Collagen | Nutrilan H, BASF Personal Care and Nutrition Gmbh |
| Hydrolyzed Keratin | Kerasol, Croda, Inc. |
| Hydrolyzed Rice Protein | Gluadin R Benz, BASF Personal Care and Nutrition Gmbh |
| Hydrolyzed Silk | Crosilk 10,000, Croda, Inc. |
| Hydrolyzed Wheat Protein | Gluadin WLM, BASF Cognis |
| Hydrolyzed Wheat Starch | Cropeptide W, Croda, Inc. |
| Hydroxyethyl Ethylcellulose | Structure Cel 4400 E, AkzoNobel Global Personal Care |
| Hydroxypropyl Guar | Jaguar C-162, Rhodia, 100% |
| Hydroxypropyltrimonium Chloride | |
| Hydroxypropyl Methylcellulose | TEGOCEL HPM 50, Evonik Industries AG, 100% |
| Hydroxypropyl Starch Phosphate | Structure 2143, AkzoNobel Global Personal Care |
| Hydroxypropyltrimonium Hydrolyzed Wheat Protein | Hydrotriticum WQ, Croda Europe, Ltd. |
| Hydroxysultaine | OriStar HSB, Orient Stars LLC |
| Inulin | Inutec H25, Beneo-Bio Based Chemicals, Div. Of Beneo-Remy NV |
| Isobutane | |
| Isopropyl Alcohol | ExxonMobilTM IPA Pharma, ExxonMobil Chemical Europe |
| Isostearamide Mipa; Glyceryl Laurate | ANTIL SPA 80, Evonik Industries AG, 100% |
| Lactic Acid | AEC Lactic Acid, A & E Connock, Perfumery & Cosmetics Ltd. |
| Lactis Proteinum | AEC Whey Protein, A & E Connock, Perfumery & Cosmetics Ltd. |
| Laureth-10 | Intrasol FA 12/18/10, Evonik Industries AG |
| Laureth-2 | Arlypon F-T, BASF Personal Care and Nutrition Gmbh |
| Laureth-23 | Tego Alkanol L 23 P, Evonik Goldschmidt GmbH |
| Laureth-4 | TEGO Alkanol L 4, Evonik Industries AG, 100% |
| Laureth-5 Carboxylic Acid | Marlowet 1072, Sasol Germany GmbH - Marl |
| Laureth-7 | Alfonic 1412-7.0 Ethoxylate, Sasol North America, Inc. |
| Laureth-9 | Marlipal 24/90, Sasol Germany GmbH - Marl |
| Lauric Acid | Prifrac 2920, Croda Europe, Ltd. |
| Lauryl Betaine | Chembetaine BW Surfactant, Lubrizol Advanced Materials, Inc. |
| Lauryl Glucoside | Plantacare 1200 UP, BASF Cognis, 50% |
| Lavandula Angustifolia (Lavender) Oil | AEC Lavender Oil, A & E Connock, Perfumery & Cosmetics Ltd. |
| Lecithin | AEC Lecithin Powder, A & E Connock, Perfumery & Cosmetics Ltd. |
| Limonene | Dipentene No. 122, Hercules Inc. |
| Linalool | Lipofresh, Lipo Chemicals, Inc. |
| Lysine | AEC Lysine, A & E Connock, Perfumery & Cosmetics Ltd. |
| Magnesium Chloride | OriStar MCL, Orient Stars LLC |
| Magnesium Nitrate | Salicat MM, Custom Ingredients, Inc. |
| Magnesium Sulfate | OriStar MS, Orient Stars LLC |
| Magnolia Officinalis Flower Extract | Magnolia Flower Extract, Xian Yuensun Biological Technology Company |
| Malic Acid | OriStar MLA, Orient Stars LLC |
| Maltodextrin | Farmal MD 10, Corn Products International |
| Malva Sylvestris (Mallow) Leaf Extract | Herbasec Mallow Leaves, Cosmetochem International AG |
| Mangifera Indica (Mango) Fruit Extract | Mango Extract, Draco Natural Products |
| Mannitol | OriStar MNT, Orient Stars LLC |
| Melissa Officinalis Extract | Melissin, Sabinsa Corporation |
| Menthol | OriStar MC, Orient Stars LLC |
| Menthyl Lactate | OriStar ML, Orient Stars LLC |
| Methyl Cocoate | CE-618, Procter & Gamble Chemicals |
| Methylchloroisothiazolinone | Microcare CB, Thor Specialties, Inc. |
| Methylchloroisothiazolinone/Methylisothia zolinone | euxyl® K 120, Schülke & Mayr GmbH /Microcare® CB/CBS;IT/ITO;ITH;ITL und andere, THOR PERSONAL CARE SAS |
| Methyldibromo Glutaronitrile | euxyl® K 400, Schülke & Mayr GmbH |
| Methylisothiazolinone | Microcare® MT, THOR PERSONAL CARE SAS |
| Methylparaben | s&m methylparaben, Schülke & Mayr GmbH |
| Microcrystalline Cellulose | Vivapur, J. Rettenmaier & Söhne |
| Mineral (Paraffinum Liquidum) Oil | Merkur White Oil Pharma; Vara 200; Vara 600, Sasol Wax GmbH |
| Mipa-Laureth Sulfate | Zetesol 2056, Zschimmer & Schwarz GmbH |
| Nacre Powder | Nano Pearl Powder, Maruei Group Co., Ltd. |
| Niacinamide | Niacinamide, USP, DSM Nutrional Products |
| Nymphaea Coerulea Flower Extract | Crodarom Blue Lotus GL, Crodarom S.A.S |
| Nymphaea Odorata Root Extract | Cosflor White Pond Lily HGS, A & E Connock, Perfumery & Cosmetics Ltd. |
| Octocrylene | Uvinul N 539 T, BASF Corporation |
| Octopirox | Octopirox, Clariant Intl. Ltd. |
| Octyldodecyl Pca | Sebumol ODPC, Zschimmer & Schwarz GmbH & Co KG |
| Olea Europaea (Olive) Fruit Oil | Cropure Olive, Croda Europe, Ltd. |
| Palmitamidopropyltrimonium Chloride | VARISOFT PATC, Evonik Industries AG, 60% |
| Palmitic Acid | Prifrac 2960, Croda Europe, Ltd. |
| Panthenol | D-Panthenol USP, BASF, 100% |
| Panthenyl Ethyl Ether | OriStar PEE, Orient Stars LLC |
| Passiflora Incarnata Seed Oil | Cegesoft PFO, BASF Personal Care and Nutrition Gmbh |
| PEG-12 Dimethicone | Silsoft 875, Momentive Performance Materials |
| PEG-120 Methyl Glucose Dioleate | ANTIL 120 Plus, Evonik Industries AG, 100% |
| PEG-14M | Polyox WSR-205, The Dow Chemical Company |
| PEG-150 Distearate | Eumulgin EO 33, BASF Personal Care and Nutrition Gmbh |
| PEG-18 | Carbowax PEG 900, The Dow Chemical Company |
| PEG-18 Castor Oil Dioleate | Marlowet CG, Sasol Germany GmbH |
| PEG-18 Glyceryl Oleate/Cocoate | ANTIL 171, Evonik Industries AG, 100% |
| PEG-20 Castor Oil | Nikkol CO-20TX, Nikko Chemicals Co., Ltd. |
| PEG-20 Glyceryl Laurate | TAGAT L 2, Evonik Industries AG, 100% |
| PEG-200 Hydrogenated Glyceryl Palmate; PEG-7 Glyceryl Cocoate | REWODERM LI S 80, Evonik Industries AG, 100% |
| PEG-3 Distearate | TEGIN D 1102, Evonik Industries AG, 100%; Cutina TS, BASF Cognis, 100% |
| PEG-3 Myristyl Ether | TEGOSOFT APM, Evonik Industries AG, 100% |
| PEG-40 Hydrogenated Castor Oil | TAGAT CH 40, Evonik Industries AG, 100% |
| PEG-7 Glyceryl Cocoate | TEGOSOFT GC, Evonik Industries AG, 100% |
| PEG-80 Sorbitan Laurate | Tween 28, Croda, Inc. |
| PEG-90 Glyceryl Isostearate | Emalex GWIS-190, Nihon Emulsion Company, Ltd. |
| PEG-90M | Polyox WSR-301, The Dow Chemical Company |
| Persea Gratissima (Avocado) Oil | Cropure Avocado, Croda Europe, Ltd. |
| Petrolatum | Merkur 115, Sasol Wax GmbH |
| Phenethyl Alcohol | Etaphen, Vevy Europe SpA |
| Phenethyl Benzoate | Finsolv Sun, Innospec Active Chemicals, LLC |
| Phenoxyethanol | S&M Phenoxyethanol, Schülke & Mayr GmbH |
| Phosphoric Acid | OriStar PPA, Orient Stars LLC |
| Piroctone Olamine | OriStar PROA, Orient Stars LLC |
| Polyglyceryl-3 Palmitate | Dermofeel PP, Dr. Straetmans |
| Polyglyceryl-4 Caprate | TEGOSOFT PC- 41, Evonik Industries AG, 100% |
| Polyglyceryl-4 Laurate | TEGO Care PL 4, Evonik Industries AG, 100% |
| Polyquaternium-10 | Polymer JR 400, Amerchol, 100% |
| Polyquaternium-11 | Dehyquart CC 11, BASF Personal Care and Nutrition Gmbh / Luviquat PQ 11 PN, BASF Corporation |
| Polyquaternium-22 | Tilamar Quat 2240, DSM Nutritional Products, Inc. / Merquat 280 Polymer, Lubrizol Advanced Materials, Inc. |
| Polyquaternium-37 | Cosmedia Ultragel 300, BASF Personal Care and Nutrition Gmbh |
| Polyquaternium-39 | Merquat Plus 3331 Polymer, Lubrizol Advanced Materials, Inc. |
| Polyquaternium-47 | Merquat 2001 N Polymer, Lubrizol Advanced Materials, Inc. |
| Polyquaternium-6 | Salcare SC30, BASF Corporation |
| Polyquaternium-7 | Merquat 550, Nalco, 100% |
| Polyquaternium-76 | Mirapol AT-1, Rhodia Novecare |
| Polysorbate 20 | TEGO SML 20, Evonik Industries AG, 100% |
| Potassium Sorbate | euxyl® K 712, Schülke & Mayr GmbH |
| PPG-12 | |
| PPG-5 Ceteth-20 | Procetyl AWS, Croda, Inc. |
| PPG-9 | Polyglycol P-425, The Dow Chemical Company |
| Propane | |
| Propylene Glycol | euxyl® K 320, Schülke & Mayr GmbH |
| Propylene Glycol (And) PEG-55 Propylene Glycol Oleate | Antil 141 Liquid, Evonik Goldschmidt GmbH |
| Propylparaben | s&m propylparaben, Schülke & Mayr GmbH |
| Prunus Amygdalus Dulcis (Sweet Almond) Extract | Cosflor White Almond HGS, A & E Connock, Perfumery & Cosmetics Ltd. |
| Prunus Amygdalus Dulcis (Sweet Almond) Oil | Cropure Almond, Croda Europe, Ltd. |
| Prunus Armeniaca (Apricot) Fruit Extract | Actiphyte of Apricot Lipo S, Active Organics, Inc. |
| Prunus Armeniaca (Apricot) Kernel Oil | AEC Apricot Kernel Oil, A & E Connock, Perfumery & Cosmetics Ltd. |
| Prunus Cerasus (Bitter Cherry) Fruit Extract | Prunus Cerasus Fruit, Kirschen Extract, Botanica GmbH |
| Pyridoxine Hcl | OriStar VB6 HCl, Orient Stars LLC |
| Pyrus Malus (Apple) Fruit Extract | AEC Apple Juice Conc, A & E Connock, Perfumery & Cosmetics Ltd. |
| Quaternium-80 | ABIL Quat 3272, Evonik Industries AG |
| Rosmarinus Officinalis (Rosemary) Leaf Extract | AEC Rosemary Extract Powder, A & E Connock, Perfumery & Cosmetics Ltd. |
| Rosmarinus Officinalis (Rosemary) Leaf Oil | AEC Rosemary Oil, A & E Connock, Perfumery & Cosmetics Ltd. |
| Saccharum Officinarum (Sugar Cane) Extract | Sugar Cane Extract, Premier Specialties Inc. |
| Salicylic Acid | OriStar SCA, Orient Stars LLC |
| Selaginella Lepidophylla Aerial Extract | Rose de Jerico Extrait Hydroglycolique, DPG, Greentech S.A |
| Silica | Aerosil 130, Evonik Degussa GmbH |
| Silicone Quaternium-18 | Silsoft Care, Momentive Performance Materials |
| Silicone Quaternium-22 | ABIL T Quat 60, Evonik Industries AG, 65% |
| Simmondsia Chinensis (Jojoba) Butter | ISO-Jojoba 35, Desert Whale Jojoba Co., Inc. |
| Simmondsia Chinensis (Jojoba) Seed Oil | AEC Jojoba Oil Refined, A & E Connock, Perfumery & Cosmetics Ltd. |
| Sodium Acetate | Essential Vital Elements, Dipta |
| Sodium Ascorbyl Phosphate | Sodium Ascorbyl Phosphate, BASF Corporation |
| Sodium Benzoate | euxyl® K 712, Schülke & Mayr GmbH |
| Sodium C14-16 Olefin Sulfonate | Bioterge AS-40 AOS, Stepan, |
| Sodium Cetearyl Sulfate | Lanette E, BASF Personal Care and Nutrition Gmbh |
| Sodium Chloride | |
| Sodium Citrate | Trisodium Citrate, Jungbunzlauer, Inc. |
| Sodium Cocoamphoacetate | REWOTERIC AM C, Evonik Industries AG, 32% |
| Sodium Cocoamphopropionate | REWOTERIC AM KSF 40, Evonik Industries AG, 40% |
| Sodium Cocoate | AEC Sodium Cocoate, A & E Connock, Perfumery & Cosmetics Ltd. |
| Sodium Cocoyl Glutamate | Plantapon ACG HC, BASF Cognis |
| Sodium Cocoyl Glycinate | Hostapon SG, Clariant; Amilite GCS-11, Ajinomoto |
| Sodium Cocoyl Isethionate | Hostapon STCI-85 P, Clariant |
| Sodium Cocoyl Sarcosinate | Crodasinic CS, Croda |
| Sodium Hydroxide | Unichem SOHYD, Universal Preserv-A-Chem, Inc. |
| Sodium Hydroxypropyl Starch Phosphate | Pure-Gel, Grain Processing Corporation |
| Sodium Isethionate | Hostapon SI, Company Clariant International Ltd, |
| Sodium Lactate | Sodium Lactate Solution About 50%, Merck KGaA/EMD Chemicals, Inc. |
| Sodium Laureth Sulfate | Texapon NSO, BASF Cognis, 28% |
| Sodium Laureth-8 Sulfate | Texapon ASV, BASF Personal Care and Nutrition Gmbh |
| Sodium Lauroamphoacetate | ColaTeric SLAA, Colonial Chemical Inc |
| Sodium Lauroamphodiacetate | Monateric 949J, Croda, Inc. |
| Sodium Lauroyl Glutamate | Corum 4063, Corum Corporation |
| Sodium Lauroyl Isethionate | Yongan SLI , Huanggang Yongan Pharmaceutical Co.,Ltd |
| Sodium Lauroyl Methyl Isethionate | Iselux, Innospec Active Chemicals |
| Sodium Lauroyl Sarcosinate | Crodasinic LS 30, Croda, 30% |
| Sodium Lauryl Sulfate | Texapon LS 35, BASF Cognis, 30% |
| Sodium Myreth Sulfate | Texapon K 14S Spez , BASF Cognis, 70% |
| Sodium Naphthalenesulfonate | NSA, Kao Corporation |
| Sodium Oleth Sulfate | Texapon ASV, BASF Personal Care and Nutrition Gmbh |
| Sodium PCA | OriStar SPCA, Orient Stars LLC |
| Sodium Salicylate | Complexe Hydroxy-Salicylique Color, BASF Beauty Care Solutions France SAS |
| Sodium Sulfate | Pro-t-action, BASF Future Business GmbH |
| Sodium Xylenesulfonate | Pilot SXS-40, Pilot Chemical Company |
| Soluble Collagen | Aquagene, BASF Beauty Care Solutions France SAS |
| Sorbitan Caprylate | Sorbon S-10, Toho Chemical Industry Co., Ltd. |
| Sorbitan Sesquicaprylate | ANTIL Soft SC, Evonik Industries AG, 100% |
| Sorbitol | OriStar SB, Orient Stars LLC |
| Stearic Acid | Pristerene 4922, Croda Europe, Ltd. |
| Styrene/Acrylates Copolymer | Acudyne HS, The Dow Chemical Company |
| Sucrose Cocoate | TEGOSOFT LSE 65 K, Evonik Industries AG, 100% |
| Tallow Acid | T-18, Procter & Gamble Chemicals |
| Taurine | OriStar TR, Orient Stars LLC |
| TEA-Dodecylbenzenesulfonate | Marlopon AT 50, Sasol Germany GmbH - Marl |
| TEA-Sulfate | Sigma-Aldrich |
| Tetrasodium Edta | Versene 100, The Dow Chemical Company |
| Tetrasodium Glutamate Diacetate | Chelest CMG-40, Chelest Corporation |
| Tilia Cordata Flower Extract | AEC Linden Blossom Extract Powder, A & E Connock, Perfumery & Cosmetics Ltd. |
| Tin Oxide | Ronastar Silver, Merck KGaA /EMD Chemicals, Inc. |
| Titanium Dioxide | Aeroxide Ti02 P25, Evonik Degussa GmbH |
| Tocopherol | euxyl® K 700, Schülke & Mayr GmbH |
| Tocopheryl Acetate | Tinoderm E, BASF Corporation |
| Trideceth-12 | Marlipal O 13/129, Sasol Germany GmbH - Marl |
| Trideceth-3 | Novel TDA-3 Ethoxylate, Sasol North America, Inc. |
| Trideceth-6 | Synperonic 13/6, Croda, Inc. |
| Trideceth-9 | Marlipal O 13/90, Sasol Germany GmbH - Marl |
| Triethanolamine | Triethanolamine Care, BASF Corporation |
| Trisodium Edta | Alcostab, Laboratoires Phytocos |
| Trisodium Ethylenediamine Disuccinate | Natriquest E30, Innospec Active Chemicals, LLC |
| Triticum Vulgare Germ Oil | Cropure Wheatgerm, Croda Europe, Ltd. |
| Xanthan Gum | Keltrol CG-SFT, CP Kelco, 100% |
| Zea Mays (Corn) Germ Oil | AEC Corn Germ Oil, A & E Connock, Perfumery & Cosmetics Ltd. |
| Zea Mays (Corn) Silk Extract | Noveact Corn Silk, Specialty Natural Products Co., Ltd. |
| Zinc Carbonate | Sigma-Aldrich |
| Zinc Pca | Zincidone, UCIB, Solabia Group |
| Zinc Pyrithione | Microcare® ZP, THOR PERSONAL CARE SAS |

## Patentansprüche

1. Kosmetische Formulierung enthaltend mindestens ein Rhamnolipid.
